# FASCICULE DE BREVET EUROPEEN

(11) **EP 3 355 861 B1**
(45) Date de publication et mention de la délivrance du brevet: **04.05.2022**
(21) Numéro de dépôt: 16775640.2
(22) Date de dépôt: 28.09.2016
(51) Int. Cl.: A61K 9/10, A45D 34/04

(54) **DISPOSITIF DE CONDITIONNEMENT D'UNE ÉMULSION**
VORRICHTUNG ZUM VERPACKEN EINER EMULSION
DEVICE FOR PACKAGING AN EMULSION

(30) Priorité: 28.09.2015 FR 1559134
(43) Date de publication de la demande: 08.08.2018
(73) Titulaire: Capsum, 13013 Marseille (FR)
(72) Inventeur: BARDON, Sébastien, 75016 Paris (FR)
(74) Mandataire: Lavoix
(86) Numéro de dépôt international: PCT/EP2016/073079
(87) Numéro de publication internationale: WO 2017/055333

(56) Documents cités:
- FR-A1- 2 971 157
- FR-A1- 2 972 367
- US-A1- 2006 141 046
- US-A1- 2014 045 949

## Description

La présente invention concerne un dispositif de conditionnement comprenant au moins une émulsion, voire une composition cosmétique, notamment parfumée.

La présente invention concerne également un procédé cosmétique non thérapeutique de maquillage et/ou de soin d'une matière kératinique et/ou pour améliorer la rétention de parfum sur une matière kératinique.

Dans le domaine cosmétique, il existe de nombreux types de compositions, notamment sous forme d'émulsions, en particulier stabilisées par des tensioactifs. Il existe également des émulsions qui peuvent comprendre un agent parfumant et qui sont stables, malgré une concentration élevée en parfum et l'absence de tensioactifs (FR 2 973 356 et US 2014/045949). Ces types de compositions sont couramment adaptés à une application topique avec les doigts de la main de l'utilisateur, ou peuvent être formulés sous forme d'un spray.

Toutefois, les consommateurs sont constamment à la recherche de produits cosmétiques faciles à utiliser et à appliquer, qui leur confèrent également de bonnes propriétés sensorielles.

En outre, la mise à disposition des consommateurs de nouveaux produits cosmétiques, notamment via des modes d'application non conventionnels en vue d'une finalité cosmétique recherchée (propriétés sensorielles, texture...), demeure un objectif constant dans ce domaine.

La présente invention vise à répondre à ces besoins.

La présente invention a donc pour but de fournir un nouveau dispositif de conditionnement permettant une application simple et rapide, tout en procurant des propriétés cosmétiques ou sensorielles intéressantes, voire améliorées, pour l'utilisateur.

Un autre but de la présente invention consiste à fournir un dispositif facile à transporter et/ou à utiliser.

La présente invention concerne donc un dispositif de conditionnement tel que défini dans la revendication 1.

L'émulsion selon l'invention comprend au moins un agent parfumant, comme décrit plus en détails ci-après, notamment au moins 2% en poids, en particulier au moins 3% en poids, de préférence de 3% à 35% en poids, en particulier de 5% à 20% en poids, mieux de 5% à 15% en poids, et plus préférentiellement de 5% à 10% en poids, d'agent(s) parfumant(s) par rapport au poids total de ladite émulsion.

Dans le cadre de l'invention, et sauf mention contraire, on entend par « dispositif de conditionnement », tout emballage permettant le conditionnement, la vente, le transport, la protection et le stockage du produit cosmétique qu'il contient. Plus particulièrement, au sens de la présente invention, le dispositif de conditionnement s'entend du produit cosmétique au sein du packaging directement à destination de l'utilisateur final.

Selon un mode de réalisation, le dispositif de conditionnement est également un dispositif d'application, notamment pour l'application de l'émulsion, sur une matière kératinique.

### Dispositif de conditionnement

### Réceptacle

Selon l'invention, le réservoir (ou chambre) peut comprendre un volume compris de 5 ml à 200 ml, de préférence de 10 ml à 100 ml, et en particulier de 15 ml à 50 ml.

Selon l'invention, l'élément comprenant au moins un orifice est choisi dans le groupe constitué d'une éponge, d'une plaque rigide percée d'au moins un trou, et de préférence percée d'une pluralité de trous, d'un filet et d'un tamis.

Selon un mode de réalisation, l'élément présentant au moins un orifice est une éponge, en particulier une éponge alvéolée. Ce type d'éponge est généralement appelé « cushion air » dans le domaine cosmétique.

Typiquement, lorsque l'élément présentant au moins un orifice est une éponge, celle-ci est imbibée (ou imprégnée) de l'émulsion selon l'invention. En d'autres termes, l'émulsion se trouve à l'intérieur de ladite éponge.

Selon un mode de réalisation, l'élément présentant au moins un orifice, lorsque figuré par une « plaque rigide percée de trou(s) », est une grille. Dans ce cas, l'orifice de la grille correspond à un trou.

Selon un mode de réalisation, lorsque l'élément présentant au moins un orifice est un tamis ou filet, celui-ci est constitué d'un croisillon de fils synthétiques formant un maillage.

La dimension des mailles du tamis peut varier de 5 µm à 600 µm, de préférence de 30 µm à 200 µm.

L'utilisation de tamis ou de grilles permet avantageusement de laisser passer l'émulsion par les interstices entre les mailles du tamis ou entre les trous de la grille.

La viscosité de l'émulsion selon l'invention est typiquement adaptée à la nature de l'élément présentant au moins un orifice. Plus particulièrement, la viscosité de l'émulsion selon l'invention est adaptée de manière à assurer un passage satisfaisant et souhaité de ladite émulsion au travers de(s) l'orifice(s), notamment lors de la mise en contact entre l'élément présentant au moins un orifice et le moyen d'application par l'utilisateur et l'application par ce dernier d'une pression sur ledit élément.

Selon un mode de réalisation, l'élément présentant au moins un orifice est donc adapté pour laisser passer une quantité de l'émulsion et donc revêtir au moins partiellement le moyen d'application en réponse à une pression exercée par l'utilisateur via le moyen d'application sur ledit élément. Ainsi, le dispositif de conditionnement selon l'invention permet de capter la juste dose de l'émulsion lorsqu'on presse le moyen d'application contre l'élément présentant au moins un orifice. L'émulsion selon l'invention peut ainsi devenir multi-fonctions car l'application, à l'aide du moyen d'application, est alors sur-mesure ; en effet, l'application de l'émulsion petit à petit permet de jouer aisément sur le degré de produit appliqué, et donc de couvrance et/ou de charge en agent(s) parfumant(s), de la plus subtile à la plus intense.

Selon l'invention, le réceptacle peut être rechargeable, c'est-à-dire qu'il est possible de recharger de l'émulsion dans le réservoir, notamment lorsqu'après plusieurs utilisations le réservoir est vide.

Selon un mode de réalisation, l'élément comprenant au moins un orifice est amovible.

Selon l'invention, lorsque l'élément présentant au moins un orifice est une éponge, celui-ci peut être rechargeable par remplacement de ladite éponge.

Le caractère rechargeable d'un dispositif selon l'invention est avantageux car écologique et permet également de moduler la teinte selon la saison.

Selon un mode de réalisation, l'élément comprenant au moins un orifice permet la fermeture d'une des faces du réservoir, notamment de la face opposée du fond du réservoir.

Selon un mode de réalisation, lorsque l'élément comprenant au moins un orifice est un tamis ou une grille, ledit élément permet de fermer le réservoir, en particulier de fermer le réceptacle.

Selon un mode de réalisation, lorsque l'élément comprenant au moins un orifice est une éponge, ledit élément est compris dans le réservoir, en particulier dans le réceptacle. Selon ce mode de réalisation, le dispositif peut en outre comprendre au moins un élément additionnel apte à fermer le réservoir, et en particulier le réceptacle.

Selon un mode de réalisation, lorsque l'élément comprenant au moins un orifice est une éponge, celle-ci peut comprendre l'émulsion selon l'invention.

Selon un mode de réalisation, lors de l'utilisation du dispositif, l'utilisateur se retrouve face à l'élément présentant au moins un orifice lorsque le dispositif est ouvert.

Selon un mode de réalisation, lorsque l'élément comprenant au moins un orifice est une plaque rigide percée d'au moins un trou, d'un tamis ou d'un filet, le dispositif selon l'invention peut comprendre en outre un dispositif de pompage de l'émulsion.

Selon l'invention, on entend par « dispositif de pompage » ou « dispositif d'aspiration », un dispositif permettant de pomper ou d'aspirer l'émulsion présente dans le réceptacle, et plus particulièrement présente dans le réservoir, vers le moyen d'application.

Selon un mode de réalisation, le dispositif de pompage est situé dans le réceptacle, et plus particulièrement entre l'élément comprenant au moins un orifice et le réservoir du réceptacle, et notamment entre l'élément comprenant au moins un orifice et l'émulsion selon l'invention.

Selon un mode de réalisation, l'élément comprenant au moins un orifice et le dispositif de pompage sont solidaires.

Le dispositif de pompage permet avantageusement le passage de l'émulsion du réservoir vers l'applicateur, en particulier par passage à travers l'(les) orifice(s) de l'élément présentant au moins un orifice, notamment lorsque l'utilisateur exerce une pression sur ledit élément comprenant au moins un orifice.

Selon un mode de réalisation, le réceptacle est constitué du réservoir contenant ladite émulsion, et dudit élément comprenant au moins un orifice.

Selon un mode de réalisation, le réceptacle est constitué du réservoir contenant ladite composition, dudit élément comprenant au moins un orifice et dudit dispositif de pompage.

### Moyen d'application

Dans le cadre de l'invention, et sauf mention contraire, on entend par « moyen d'application » ou « élément applicateur » ou « organe d'application » ou « applicateur », un élément permettant de prélever au moins une partie de l'émulsion comprise dans le réservoir et de l'appliquer sur une surface, notamment sur une matière kératinique.

Selon l'invention, le moyen d'application est choisi dans le groupe constitué d'une brosse, d'une houppe, d'une houppette, d'une mousse, d'un bâtonnet, par exemple d'un bâtonnet en silicone, et d'un pinceau. L'applicateur peut posséder toute forme, et notamment toute section. Par exemple, l'applicateur peut présenter une section transversale circulaire, ovale, ou polygonale, par exemple carrée ou triangulaire.

Selon un mode de réalisation, l'applicateur est une mousse, en particulier sous forme de disque.

Dans la présente invention, la mousse peut être également désignée par « coussin », « coussinet » ou encore par « pad ».

Selon l'invention, la mousse peut être synthétique.

Selon un mode de réalisation, l'applicateur est en mousse, qui peut être réticulée ou non, et qui peut comprendre des alvéoles ouvertes ou fermées. De préférence, l'applicateur comprend une couche de mousse de polyuréthane réticulée.

La mousse peut être choisie dans le groupe constitué d'une mousse de chlorure de polyvinyle (PVC), de polyuréthanne, de polyéther, de polyester ; d'un élastomère de type SBR (Synthetic Butadiène Rubber), NBR, silicone, nitrile ; et de leurs mélanges.

Selon un autre mode de réalisation, l'applicateur est une mousse de Rubycell à alvéoles dites fermées, non-réticulée, ayant un diamètre de pores compris de 50 à 500 pores par inch. Rubycell est notamment commercialisée par la société Toyo Polymer Co. Ltd.

De préférence, la mousse est une mousse d'un mélange de polyuréthane et de Rubycell.

Selon l'invention, l'applicateur peut être une mousse constituée de l'un des matériaux floqués suivants : le nylon, les polyesters, les acryliques, le coton, et leurs mélanges.

Selon un mode de réalisation, l'applicateur est une mousse constituée de l'un des matériaux suivants : polyester, chlorure de polyvinyle (PVC), PVA (polyvinyl alcohol), polyuréthane, polyéther, élastomère de type SBR (Synthetic Butadiène Rubber), de type NBR (acrylnitrile butadiene rubber), de type silicone ou de type nitrile, caoutchouc naturel ou synthétique (notamment à alvéoles fermées), polyoléfine, EDPM (Ethylene, Propylene, Diene, Monomer), cellulose, ou leurs mélanges.

Selon un mode de réalisation, l'applicateur est une mousse appelée Yukilon, commercialisé par la société Yukigaya Chemical Industries Co., Ltd., Tokyo, Japan.

Selon un mode de réalisation, l'applicateur comprend une ou plusieurs couches de polyéthylène, de polyuréthane ou polypropolène, de Latex synthétique ou naturel, d'EDPM, de caoutchouc, de styrène, de nylon, de papier revêtu, de polyoléfine, de plastiques constitués de nylons ou polyesters, de Téflon, de PVA ou PVC.

Selon un mode de réalisation, l'applicateur est en coton, notamment en coton velours tissé, en nylon tissé, en acrylique, en laine, en flanelle, en polyester, en coton, ou en leurs mélanges.

Selon un mode de réalisation, l'applicateur est en tissu tricoté de coton, de polyester, d'acrylique, de nylon, de flanelle, de feutre, ou de leurs mélanges.

Selon un mode de réalisation, la mousse est microporeuse, et comprend notamment des pores de diamètre compris entre 10 et 500 microns.

Selon un mode de réalisation, l'applicateur peut être indépendant de tout support, notamment lorsqu'il s'agit d'une mousse.

Selon un mode de réalisation, l'applicateur est solidaire d'un support permettant avantageusement à l'utilisateur la préhension de l'applicateur sans contact avec celui-ci. Ceci évite avantageusement à l'utilisateur un contact direct avec l'émulsion à appliquer, et donc de se souiller les doigts avec l'émulsion à appliquer.

Selon un mode de réalisation, en exerçant une pression de l'applicateur sur l'élément présentant au moins un orifice du réceptacle, l'applicateur se comprime au moins partiellement et l'élément présentant au moins un orifice se déforme réduisant ainsi le volume disponible pour l'émulsion dans le réservoir, de sorte qu'au moins une partie de l'émulsion peut sortir par l'orifice et ainsi venir au contact de la surface dudit applicateur. Ainsi, l'utilisateur peut prélever une certaine dose de l'émulsion sur l'applicateur pour ensuite la déposer sur la matière kératinique.

Selon un mode de réalisation, lorsque le dispositif de conditionnement selon l'invention comprend un dispositif de pompage tel que décrit ci-dessus, en exerçant une pression de l'applicateur sur l'élément présentant au moins un orifice du réceptacle, une partie de l'émulsion peut passer du réservoir au moyen d'application via le dispositif de pompage.

Typiquement, la pression exercée par l'utilisateur peut être une pression effectuée par tamponnement de l'applicateur sur l'élément présentant au moins un orifice.

Selon l'invention, l'applicateur peut être logé au-dessus du réservoir, voire du réceptacle, lorsque le dispositif est fermé, et donc en l'absence d'utilisation.

### Dispositif

Le dispositif de conditionnement selon l'invention peut comporter au moins un organe de fermeture, destiné à fermer le réceptacle, de préférence de manière étanche en l'absence d'utilisation.

Selon un mode de réalisation, l'applicateur est logé sur une face de l'organe de fermeture. Avantageusement, l'organe de fermeture permet de séparer l'applicateur du réceptacle comprenant l'émulsion, ce qui permet d'éviter de souiller l'applicateur lors de l'absence d'utilisation du dispositif.

Le dispositif peut comporter un boitier muni d'un couvercle et d'un corps. De préférence, le boitier est de forme cylindrique.

De préférence, le dispositif est un poudrier.

Le dispositif peut également comprendre un miroir disposé sur la face interne du couvercle. De préférence, le miroir couvre la totalité de la face interne du couvercle, pour offrir une surface de vision la plus confortable possible pour l'utilisateur.

Selon un mode de réalisation, l'utilisateur dispose d'un dispositif se présentant, lorsqu'il est en configuration d'utilisation, sous la forme d'un boitier compact avec deux faces principales, l'une comportant le réceptacle avec l'émulsion, et la face opposée comportant le miroir.

Le dispositif de conditionnement selon l'invention est typiquement assimilable à un produit de type « cushion cream » ou « cushion foundation » ou « compact cushion » connus pour comprendre un réceptacle comprenant une éponge alvéolée (dit « air cushion ») imbibée d'une crème teintée qui unifie la peau, l'hydrate et la protège des rayons ultraviolets. Typiquement, la crème s'applique à l'aide d'un petit coussin (ou « Pad » ou « disque en mousse ») que l'utilisateur vient tamponner contre le poudrier.

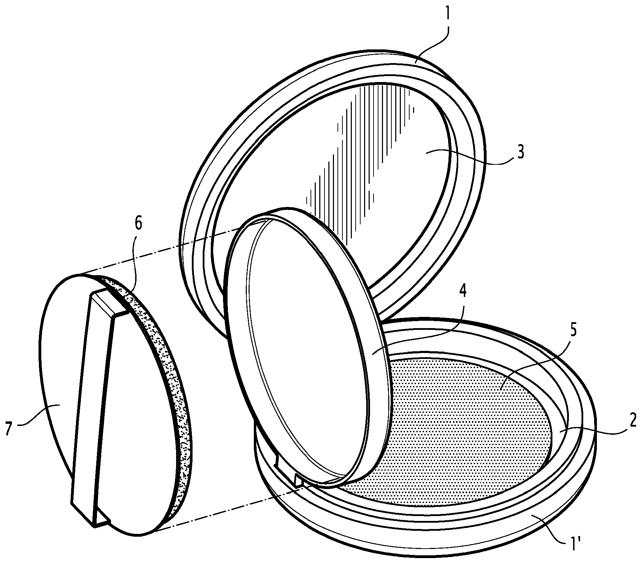

La figure 1 décrit un mode préféré de dispositif de conditionnement selon l'invention, en mode ouvert (pour utilisation). En particulier, ce dispositif, de forme ronde, comprend un boîtier (couvercle (1) et corps (1')), un miroir (3) situé sur la face interne du couvercle (1) et un réceptacle comprenant un élément présentant au moins un orifice (5), tel qu'une éponge. De plus, le dispositif selon la figure 1 comprend un organe de fermeture (4) qui est en position ouverte, et qui peut contenir l'applicateur (6) comprenant un support (7) facilitant sa préhension.

Le dispositif de conditionnement selon l'invention peut être adapté pour être porté sur soi par un utilisateur, par exemple dans un sac à main. En effet, le dispositif de conditionnement selon l'invention présente avantageusement un caractère transportable ou « nomade » et est conçu pour être saisi à/ou dans la main. Le dispositif selon l'invention est donc avantageux car petit et facile à transporter, ce qui permet à l'utilisateur de le transporter dans son sac à main par exemple ou même dans une poche de vêtement.

L'applicateur du dispositif permet avantageusement une application facile de l'émulsion. En particulier, un applicateur sous forme de mousse permet avantageusement une application agréable et permet de réguler la dose à prélever et à appliquer. De plus, l'application régulée de l'émulsion permet avantageusement de jouer sur les degrés de couvrance et/ou de charge d'agent(s) parfumant(s), de la plus subtile à la plus intense.

La présence d'un applicateur dans le dispositif permet avantageusement d'éviter tout contact entre les doigts de l'utilisateur et l'émulsion à appliquer.

Par ailleurs, il a été avantageusement observé que l'utilisation d'un dispositif selon l'invention permet d'améliorer les propriétés sensorielles ressenties par l'utilisateur lors de l'application de l'émulsion sur une matière kératinique, notamment en termes d'hydratation, de sensation de fraîcheur et de légèreté (bonne couvrance tout en restant léger sur la peau).

En outre, la mise en œuvre d'un produit de type « cushion cream » est une alternative inédite proposée aux utilisateurs pour l'application d'un parfum (ou composition parfumante). Qui plus est, une meilleure rémanence de l'effet olfactif conféré par l'agent(s) parfumant(s) et une puissance olfactive améliorée ont pu avantageusement être observées lors de l'application d'une émulsion selon l'invention comprenant au moins un agent parfumant au moyen du dispositif de conditionnement selon l'invention.

### Emulsion

Selon l'invention, l'émulsion comprend une phase continue et une phase dispersée sous forme de gouttes, lesdites gouttes comprenant une écorce formée d'au moins un polymère anionique et d'au moins un polymère cationique.

Selon un mode de réalisation, l'émulsion est de type eau-dans-huile ou de type huile-dans-eau.

Selon un mode de réalisation préféré, l'émulsion est de type huile-dans-eau et comprend ainsi une phase aqueuse continue et une phase grasse dispersée sous forme de gouttes, lesdites gouttes comprenant une écorce formée d'au moins un polymère anionique et d'au moins un polymère cationique.

Dans le cadre de la présente invention, l'émulsion susmentionnée peut être désignée indifféremment par le terme "dispersions".

Selon un mode de réalisation, une émulsion selon l'invention ne comprend pas de tensioactif. Elle se différencie donc des compositions cosmétiques usuelles, notamment sous forme d'émulsion.

Les émulsions selon l'invention présentent un intérêt particulier en ce qui concerne la texture en se différenciant des émulsions « classiques » stabilisées par des tensioactifs.

En effet, les émulsions selon l'invention se caractérisent par une texture unique, légère et volubile, procurant une application en deux temps. Plus particulièrement, les émulsions selon l'invention s'étalent facilement sur la peau. Les premiers instants d'application sont très aqueux avec un effet cassant marqué. Puis, le ressenti évolue vers un voile huileux qui s'estompe pour laisser une peau légère et hydratée.

Selon l'invention, le pH de l'émulsion est typiquement compris entre 5,5 et 8,0.

Selon un mode de réalisation, une émulsion selon l'invention est préparée par mise en œuvre d'un procédé « non-microfluidique », à savoir par simple émulsification. Selon ce mode de réalisation, la taille des gouttes de la phase dispersée est inférieure à 500 µm, voire inférieure à 200 µm. Préférentiellement, la taille des gouttes est comprise entre 0,5 µm à 50 µm, de préférence entre 1 µm et 20 µm.

Selon ce mode de réalisation, la présente invention permet de disposer de gouttes de taille réduite, notamment par rapport à des gouttes obtenues par un procédé microfluidique. Cette petite taille de gouttes va avoir un effet sur la texture. En effet, une émulsion selon l'invention, formée de gouttes finement dispersées, présente des qualités d'onctuosité améliorée.

Dans le cadre de la présente invention, le terme "taille" désigne le diamètre, notamment le diamètre moyen, des gouttes.

### Viscosité

La viscosité des émulsions selon l'invention peut varier de façon importante ce qui permet donc d'obtenir des textures variées.

Selon un mode de réalisation, l'émulsion selon l'invention a une viscosité comprise de 1 mPa.s à 500 000 mPa.s, de préférence de 10 à 300 000 mPa.s, et mieux de 1 000 mPa.s à 100 000 mPa.s, telle que mesurée à 25°C.

La viscosité est mesurée à température ambiante, par exemple T=25°C ± 2°C et à pression ambiante, par exemple 1013 mbar, par la méthode décrite ci-après.

On utilise un viscosimètre de type Brookfield, typiquement un viscosimètre numérique Brookfield RVDV-E (couple de torsion du ressort de 7187,0 dyne-cm), qui est un viscosimètre rotationnel à vitesse imposée muni d'un mobile (désigné par le terme anglais « Spindle »). Une vitesse est imposée au mobile en rotation et la mesure du couple exercé sur le mobile permet de déterminer la viscosité en connaissant les paramètres de géométrie/forme du mobile utilisé.

On utilise par exemple un mobile de taille No. 04 (référence Brookfield : RV4). Le taux de cisaillement correspondant à la mesure de la viscosité est défini par le mobile utilisé et la vitesse de rotation de celui-ci.

La mesure de viscosité est effectuée sur 1 minute à température ambiante (T=25°C ± 2°C). On place environ 150 g de solution dans un bécher de 250 ml de volume, ayant un diamètre d'environ 7 cm de façon à ce que la hauteur du volume occupée par les 150 g de solution soit suffisante pour arriver à la jauge marquée sur le mobile. Ensuite, on démarre le viscosimètre sur une vitesse de 10 tours/min et on attend que la valeur affichée sur l'écran soit stable. Cette mesure donne la viscosité du fluide testé, telle que mentionnée dans le cadre de la présente invention.

### Phase grasse

Selon l'invention, les émulsions peuvent comprendre une phase grasse, notamment dispersée, sous forme de gouttes.

Selon l'invention, la phase grasse peut comprendre au moins une huile H1 dans laquelle le polymère cationique est soluble. En effet, l'émulsion selon l'invention peut comprendre au moins une huile compatible avec le polymère cationique. L'huile H1 correspond donc à un bon solvant du polymère cationique.

On entend par « huile » un corps gras liquide à la température ambiante (25°C).

Comme huiles H1 utilisables dans une émulsion selon l'invention, on peut citer par exemple :
- les huiles hydrocarbonées d'origine animale, telles que le perhydrosqualène et le squalane ;
- les esters et les éthers de synthèse, notamment d'acides gras, comme les huiles de formules R₁COOR₂ et R₁OR₂ dans laquelle R₁ représente le reste d'un acide gras en C₈ à C₂₉, et R₂ représente une chaîne hydrocarbonée, ramifiée ou non, en C₃ à C₃₀, comme par exemple l'huile de Purcellin, l'isononanoate d'isononyle, le néopentanoate d'isodécyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle (Prisorine 3631) ;

- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de Parléam ;
- les huiles de silicone, comme par exemple les polyméthylsiloxanes (PDMS) volatiles ou non à chaine siliconée linéaire ou cyclique, liquides ou pâteux à température ambiante, notamment les cyclopolydiméthylsiloxanes (cyclométhicones) telles que la cyclohexasiloxane et la cyclopentasiloxane ; les polydiméthylsiloxanes (ou diméthicones) comportant des groupements alkyle, alcoxy ou phényle, pendant ou en bout de chaine siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- les alcools gras ayant de 8 à 26 atomes de carbone, comme l'alcool cétylique, l'alcool stéarylique et leur mélange (alcool cétylstéarylique), ou encore l'octyldodécanol ;
- les huiles fluorées partiellement hydrocarbonées et/ou siliconées comme celles décrites dans le document JP-A-2-295912 ;
- et leurs mélanges.

Selon un mode de réalisation, l'huile H1 est choisie parmi les esters de formule R₁COOR₂, dans laquelle R₁ représente le reste d'un acide gras en C₈ à C₂₉, et R₂ représente une chaîne hydrocarbonée, ramifiée ou non, en C₃ à C₃₀.

Selon un mode de réalisation, l'huile H1 est choisie parmi les alcools gras ayant de 8 à 26 atomes de carbone.

Selon un mode de réalisation, l'huile H1 est choisie parmi les huiles de silicone, comme par exemple les polydiméthylsiloxanes (PDMS).

Selon un mode de réalisation, l'huile H1 est choisie parmi les huiles hydrocarbonées ayant de 8 à 16 atomes de carbone, et notamment les alcanes ramifiés en C₈-C₁₆ (appelées aussi isoparaffines ou isoalcanes), comme l'isododécane (encore appelé 2,2,4,4,6-pentaméthylheptane), l'isodécane, l'isohexadécane, et, par exemple, les huiles vendues sous les noms commerciaux d'lsopars^{®}ou de Permethyls^{®}.

Selon un mode de réalisation préféré, l'huile H1 est choisie dans le groupe constitué de l'isononanoate d'isononyle, de la diméthicone, de l'isohexadécane, du polydiméthylsiloxane, de l'octyldodécanol, du néopentanoate d'isodécyle et de leurs mélanges.

De préférence, l'huile H1 est l'isononanoate d'isononyle.

Selon un mode de réalisation, l'huile H1 n'est pas une huile végétale.

Selon un mode de réalisation, l'huile H1 n'est pas figurée par le polydiméthylsiloxane (PDMS), et de préférence n'est pas une huile de silicone.

Selon un autre mode de réalisation, la phase grasse des gouttes ne comprend pas de polydiméthylsiloxane (PDMS), et de préférence ne comprend pas d'huile de silicone.

Selon un mode de réalisation préféré, une émulsion selon l'invention comprend au moins 1% en poids d'huile(s) H1, de préférence d'isononanoate d'isononyle, par rapport au poids total de ladite émulsion.

Selon un mode de réalisation, la teneur en huile(s) H1 dans la phase grasse est comprise entre 1% et 99,99%, de préférence entre 20% et 90%, et en particulier entre 50% et 80%, en poids par rapport au poids total de ladite phase grasse.

Selon un mode de réalisation, la phase grasse des émulsions de l'invention peut comprendre en outre au moins une huile hydrocarbonée d'origine végétale H2. La phase grasse peut comprendre plusieurs huiles H2.

Comme huiles végétales H2, on peut notamment citer les triglycérides liquides d'acides gras en C₄-C₁₀ comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux commercialisés par la société Stearineries Dubois ou ceux disponibles sous les dénominations commerciales « Miglyol 810 », « Miglyol 812 » et « Miglyol 818 » par la société Dynamit Nobel, l'huile de jojoba, l'huile de beurre de karité, et leurs mélanges.

De préférence, l'huile H2 est choisie parmi les huiles riches en acides gras polyinsaturés.

On entend par "acide gras insaturé" au sens de la présente invention, un acide gras comprenant au moins une double liaison. Il s'agit plus particulièrement d'acides gras à longues chaînes, c'est-à-dire pouvant posséder plus de 14 atomes de carbone. Les acides gras insaturés peuvent être sous forme acide, ou sous forme de sel, comme par exemple leur sel de calcium, ou encore sous forme de dérivés, notamment d'ester(s) d'acide(s) gras.

De préférence, l'huile H2 est choisie parmi les huiles riches en acides gras à longues chaînes, c'est-à-dire pouvant posséder plus de 14 atomes de carbone, et mieux en acides gras insaturés comportant de 18 à 22 atomes de carbone, en particulier en acides gras ω-3 et ω-6. Ainsi, avantageusement, les huiles végétales sont choisies parmi les huiles d'onagre, de bourrache, de pépins de cassis, de chanvre, de noix, de soja, de tournesol, de germes de blé, de fénugrec, de rosier muscat, d'échium, d'argan, de baobab, de son de riz, de sésame, d'amande, de noisette, de chia, de lin, d'olive, d'avocat, de carthame, de coriandre, de colza (notamment Brassica naptus), et leurs mélanges.

De préférence, l'huile H2 est choisie parmi les huiles mates et non brillantes. Peut notamment être citée à ce titre l'huile de Moringa.

Selon un mode de réalisation, la teneur en huile(s) H2 dans la phase grasse est comprise entre 0% et 40%, de préférence entre 0,1% et 25%, et en particulier entre 1% et 20%, en poids par rapport au poids total de ladite phase grasse.

Selon un mode de réalisation, le ratio massique entre la quantité d'huile(s) H1 et la quantité d'huile(s) H2 va de 0,025 à 99,99, de préférence de 0,8 à 90, et en particulier de 2,5 à 80.

La phase grasse peut comprendre en outre au moins une autre huile différente des huiles H1 et H2.

Une émulsion huile-dans-eau selon l'invention peut comprendre de 0,0001 % à 50%, de préférence de 0,1% à 40%, et mieux de 1% à 25%, en poids d'huile(s) par rapport au poids total de ladite émulsion.

Selon un mode de réalisation, la phase grasse d'une émulsion selon l'invention peut comprendre au moins un corps gras solides à température et pression ambiante, notamment choisi parmi les cires, les corps gras pâteux, les beurres, et leurs mélanges.

### Cire(s)

Par « cire », on entend au sens de l'invention, un composé lipophile, solide à température ambiante (25°C), à changement d'état solide/liquide réversible, ayant un point de fusion supérieur ou égal à 30 °C pouvant aller jusqu'à 120°C. Le point de fusion peut être mesuré selon le protocole décrit dans FR 15 58849.

Les cires susceptibles d'être utilisées dans une émulsion selon l'invention sont choisies parmi les cires, solides, déformables ou non à température ambiante, d'origine animale, végétale, minérale ou de synthèse et leurs mélanges.

On peut notamment utiliser les cires hydrocarbonées comme la cire d'abeilles, la cire de lanoline, et les cires d'insectes de Chine; la cire de riz, la cire de Carnauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines et l'ozokérite; les cires de polyéthylène, les cires obtenues par la synthèse de Fisher-Tropsch et les copolymères cireux ainsi que leurs esters, et leurs mélanges.

On peut aussi citer les cires obtenues par hydrogénation catalytique d'huiles animales ou végétales ayant des chaînes grasses, linéaires ou ramifiées, en C₃-C₃₂.

Parmi celles-ci, on peut notamment citer l'huile de jojoba hydrogénée, l'huile de tournesol hydrogénée, l'huile de ricin hydrogénée, l'huile de coprah hydrogénée et l'huile de lanoline hydrogénée, le tétrastéarate de di-(triméthylol-1,1,1 propane) vendu sous la dénomination « HEST 2T-4S » par la société HETERENE, le tétrabéhénate de di-(triméthylol-1,1,1 propane) vendue sous la dénomination HEST 2T-4B par la société HETERENE.

On peut également utiliser les cires obtenues par transestérification et hydrogénation d'huiles végétales, telles que l'huile de ricin ou d'olive, comme les cires vendues sous les dénominations de Phytowax ricin 16L64^{®} et 22L73^{®} et Phytowax Olive 18L57 par la société SOPHIM. De telles cires sont décrites dans la demande FR-A- 2792190.

On peut aussi utiliser des cires siliconées qui peuvent être avantageusement des polysiloxanes substitués, de préférence à bas point de fusion.

Parmi les cires de silicones commerciales de ce type, on peut citer notamment celles vendues sous les dénominations Abilwax 9800, 9801 ou 9810 (GOLDSCHMIDT), KF910 et KF7002 (SHIN ETSU), ou 176-1118-3 et 176-11481 (GENERAL ELECTRIC).

Les cires de silicone utilisables peuvent également être des alkyl ou alcoxydiméthicones tels que les produits commerciaux suivants : Abilwax 2428, 2434 et 2440 (GOLDSCHMIDT), ou VP 1622 et VP 1621 (WACKER), ainsi que les (C₂₀-C₆₀) alkyldiméthicones, en particulier les (C₃₀-C₄₅) alkyldiméthicones comme la cire siliconée vendue sous la dénomination SF-1642 par la société GE-Bayer Silicones.

On peut également utiliser des cires hydrocarbonées modifiées par des groupements siliconés ou fluorés comme par exemple : siliconyl candelilla, siliconyl beeswax et Fluorobeeswax de Koster Keunen.

Les cires peuvent également être choisies parmi les cires fluorées.

### Beurre(s) ou corps gras pâteux

Par « beurre » (également appelé « corps gras pâteux ») au sens de la présente invention, on entend un composé gras lipophile à changement d'état solide/liquide réversible et comportant à la température de 25°C une fraction liquide et une fraction solide, et à pression atmosphérique (760 mm Hg).

Le corps gras pâteux ou beurre peut être choisi parmi les composés synthétiques et les composés d'origine végétale. Un corps gras pâteux peut être obtenu par synthèse à partir de produits de départ d'origine végétale.

Le corps gras pâteux est avantageusement choisi parmi :
- la lanoline et ses dérivés tels que l'alcool de lanoline, les lanolines oxyéthylénées, la lanoline acétylée, les esters de lanoline tels que le lanolate d'isopropyle, les lanolines oxypropylénées,
- les composés siliconés polymères ou non-polymères comme les polydiméthysiloxanes de masses moléculaires élevées, les polydiméthysiloxanes à chaînes latérales du type alkyle ou alcoxy ayant de 8 à 24 atomes de carbone, notamment les stéaryl diméthicones,
- les composés fluorés polymères ou non-polymères,
- les polymères vinyliques, notamment
- les homopolymères d'oléfines,
- les copolymères d'oléfines,
- les homopolymères et copolymères de diènes hydrogénés,
- les oligomères linéaires ou ramifiés, homo ou copolymères de (méth)acrylates d'alkyle ayant de préférence un groupement alkyle en C₈-C₃₀,
- les oligomères homo et copolymères d'esters vinyliques ayant des groupements alkyles en C₈-C₃₀,
- les oligomères homo et copolymères de vinyléthers ayant des groupements alkyles en C₈-C₃₀,
- les polyéthers liposolubles résultant de la polyéthérification entre un ou plusieurs diols en C₂-C₁₀₀, de préférence en C₂-C₅₀,
- les esters et les polyesters, et
- leurs mélanges.

Comme beurres d'origine végétale, on peut citer ceux décrit dans Ullmann's Encyclopedia of Industrial Chemistry (« Fats and Fatty Oils», A. Thomas, publié le 15/06/2000, D01: 10.1002/14356007.a10_173, point 13.2.2.2. Shea Butter, Borneo Tallow, and Related Fats (Vegetable Butters)). On peut citer plus particulièrement les triglycérides en C₁₀-C₁₈ (nom INCI : C10-18 Triglycérides) comportant à la température de 25°C et à pression atmosphérique (760 mm Hg) une fraction liquide et une fraction solide, le beurre de karité, le beurre de Karité Nilotica *(Butyrospermum parkii*), le beurre de Galam, (*Butyrospermum parkii*), le beurre ou graisse de Bornéo ou tengkawang tallow) *(Shorea stenoptera),* beurre de Shorea, beurre d'Illipé , beurre de Madhuca ou Bassia Madhuca longifolia, beurre de mowrah *(Madhuca Latifolia*), beurre de Katiau *(Madhuca mottleyana*), le beurre de Phulwara (M. *butyracea),* le beurre de mangue *(Mangifera indica),* le beurre de Murumuru *(Astrocatyum murumuru),* le beurre de Kokum (*Garcinia Indica*), le beurre d'Ucuuba *(Virola sebifera),* le beurre de Tucuma, le beurre de Painya (Kpangnan) *(Pentadesma butyracea),* le beurre de café *(Coffea arabica),* le beurre d'abricot *(Prunus Armeniaca*), le beurre de Macadamia *(Macadamia Temifolia*), le beurre de pépin de raisin *(Vitis vinifera),* le beurre d'avocat *(Persea gratissima),* le beurre d'olives (*Olea europaea),* le beurre d'amande douce *(Prunus amygdalus dulcis*), le beurre de cacao *(Theobroma cacao)* et le beurre de tournesol, le beurre sous le nom INCI Astrocaryum Murumuru Seed Butter, le beurre sous le nom INCI Theobroma Grandiflorum Seed Butter, et le beurre sous le nom INCI Irvingia Gabonensis Kernel Butter, les esters de jojoba (mélange de cire et d'huile de jojoba hydrogénée)(nom INCI : Jojoba esters) et les esters éthyliques de beurre de karité (nom INCI : Shea butter ethyl esters), et leurs mélanges.

De préférence, une émulsion selon l'invention peut comprendre de 0% à 98,99% en poids, de préférence de 0,5% à 70% en poids, en particulier de 1% à 30% en poids, et mieux de 1% à 20% en poids, de corps gras solide(s) par rapport au poids total de la phase grasse.

Selon un mode de réalisation où l'émulsion selon l'invention comprend au moins un agent parfumant, la phase grasse peut être dénuée d'huile et/ou de corps gras solide. En effet, certains agents parfumant, outre leur action parfumante, agissent également comme solvant du polymère cationique, notamment l'amodiméthicone.

### Ecorce des gouttes

Comme mentionné précédemment, les gouttes selon l'invention sont entourées d'une écorce (également désignée par le terme « membrane »).

Selon l'invention, les gouttes obtenues peuvent présenter une écorce très fine, notamment d'épaisseur inférieure à 1% du diamètre des gouttes.

L'épaisseur de l'écorce est ainsi de préférence inférieure à 1 µm et est trop faible pour être mesurée par des méthodes optiques.

Selon un mode de réalisation, l'épaisseur de l'écorce des gouttes est inférieure à 1 000 nm, notamment comprise de 1 à 500 nm, de préférence inférieure à 100 nm, avantageusement inférieure à 50 nm, préférentiellement inférieure à 10 nm.

La mesure de l'épaisseur de l'écorce des gouttes de l'invention peut être effectuée par la méthode de diffusion de neutrons aux petits angles (Small-Angle X-ray Scattering), telle que mise en œuvre dans Sato et al. J. Chem. Phys. 111, 1393-1401 (2007).

Pour cela, les gouttes sont produites en utilisant de l'eau deutérée, puis sont lavées trois fois avec une huile deutérée, comme par exemple une huile deutérée de type hydrocarboné (octane, dodécane, hexadécane).

Après lavage, les gouttes sont ensuite transférées dans la cellule de Neutrons afin de déterminer le spectre I(q) ; q étant le vecteur d'onde.

A partir de ce spectre, on applique les traitements analytiques classiques (REF) afin de déterminer l'épaisseur de l'écorce hydrogénée (non deutérée).

Selon un mode de réalisation, l'écorce entourant les gouttes de la phase dispersée est rigidifiée, ce qui confère notamment une bonne résistance aux gouttes et diminue, voire empêche, leur coalescence.

Cette écorce est typiquement formée par coacervation, c'est-à-dire par précipitation de polymères chargés de charges opposées. Au sein d'un coacervat, les liaisons liant les polymères chargés entre eux sont de type ionique, et sont généralement plus fortes que des liaisons présentes au sein d'une membrane de type tensioactif.

L'écorce est formée par coacervation d'au moins deux polymères chargés de polarité opposée (ou polyélectrolyte) et de préférence en présence d'un premier polymère, de type cationique, et d'un deuxième polymère, différent du premier polymère, de type anionique. Ces deux polymères jouent le rôle d'agents de rigidification de la membrane.

La formation du coacervat entre ces deux polymères est généralement provoquée par une modification des conditions du milieu réactionnel (température, pH, concentration en réactifs, etc.). La réaction de coacervation résulte de la neutralisation de ces deux polymères chargés de polarités opposées et permet la formation d'une structure membranaire par interactions électrostatiques entre le polymère anionique et le polymère cationique. La membrane ainsi formée autour de chaque goutte forme typiquement une écorce qui encapsule totalement le cœur de la goutte et isole ainsi le cœur de la goutte de la phase aqueuse continue.

### Polymère anionique

Dans le cadre de la présente invention, on entend par "polymère anionique" (ou "polymère de type anionique") un polymère comportant des fonctions chimiques de type anionique. On peut aussi parler de polyélectrolyte anionique.

Par "fonction chimique de type anionique", on entend une fonction chimique AH capable de céder un proton pour donner une fonction A⁻. Selon les conditions du milieu dans lequel il se trouve, le polymère de type anionique comporte donc des fonctions chimiques sous forme AH, ou bien sous forme de sa base conjuguée A⁻.

Comme exemple de fonctions chimiques de type anionique, on peut citer les fonctions acides carboxyliques -COOH, éventuellement présentes sous forme d'anion carboxylate -COO-.

Comme exemple de polymère de type anionique, on peut citer tout polymère formé par la polymérisation de monomères dont au moins une partie porte des fonctions chimiques de type anionique, tel que des fonctions acide carboxylique. De tels monomères sont par exemple l'acide acrylique, l'acide maléique, ou tout monomère éthyléniquement insaturé comportant au moins une fonction acide carboxylique. Il peut par exemple s'agir de polymère anionique comprenant des unités monomères comportant au moins une fonction chimique de type acide carboxylique.

De préférence, le polymère anionique est hydrophile, c'est-à-dire soluble ou dispersible dans l'eau.

Selon un mode de réalisation, la phase continue, de préférence la phase aqueuse continue, comprend au moins un polymère anionique.

Parmi les exemples de polymère anionique appropriés à la mise en œuvre de l'invention, on peut citer les copolymères d'acide acrylique ou d'acide maléique et d'autres monomères, tels que l'acrylamide, les acrylates d'alkyle, les acrylates d'alkyle en C₅-C₈, les acrylates d'alkyle en C₁₀-C₃₀, les méthacrylates d'alkyle en C₁₂-C₂₂, les méthacrylates méthoxypolyéthylèneglycol, les acrylates d'hydroxyester, les acrylates crosspolymères et leurs mélanges.

Selon un mode de réalisation, le polymère anionique selon l'invention est un carbomère ou un copolymère réticulé acrylates/C₁₀₋₃₀ alkyl acrylate. De préférence, le polymère anionique selon l'invention est un carbomère.

Selon un mode de réalisation, l'écorce des gouttes comprend au moins un polymère anionique, tel que par exemple un carbomère.

Dans le cadre de l'invention, et sauf mention contraire, on entend par "carbomère", un homopolymère éventuellement réticulé, issu de la polymérisation de l'acide acrylique. Il s'agit donc d'un poly(acide acrylique) éventuellement réticulé.

Parmi les carbomères de l'invention, on peut citer ceux commercialisés sous les noms Tego^{®}Carbomer 340FD de Evonik, Carbopol^{®} 981 de Lubrizol, Carbopol ETD 2050 de Lubrizol, ou encore Carbopol Ultrez 10 de Lubrizol.

Selon un mode de réalisation, on entend par "carbomère" ou "carbomer" ou "Carbopol^{®}" un polymère d'acide acrylique de haut poids moléculaire réticulé avec du sucrose allylique ou des éthers allyliques de pentaérythritol (handbook of Pharmaceutical Excipients, 5eme Edition, plll). Par exemple, il s'agit du Carbopol^{®} 910, du Carbopol^{®} 934, Carbopol^{®} 934P, du Carbopol^{®} 940, du Carbopol^{®} 941, du Carbopol^{®} 71G, du Carbopol^{®} 980, du Carbopol^{®} 971 P ou du Carbopol^{®}974P. Selon un mode de réalisation, la viscosité dudit carbomère est comprise entre 4 000 et 60 000 cP à 0,5% w/w.

Les carbomères ont d'autres dénominations : acides polyacryliques, polymères carboxyvinyliques ou carboxy polyéthylènes.

Selon l'invention, la dispersion susmentionnée peut comprendre de 0,01% à 5%, de préférence de 0,05% à 2%, et préférentiellement de 0,10% à 0,5%, en poids de polymère(s) anionique(s), notamment de carbomère(s), par rapport au poids total de ladite dispersion.

### Polymère cationique

Les gouttes, et notamment l'écorce desdites gouttes, comprennent un polymère de type cationique. Elles peuvent également comprendre plusieurs polymères de type cationique. Ce polymère cationique est celui mentionné ci-dessus qui forme l'écorce par coacervation avec le polymère anionique.

Dans le cadre de la présente demande, et sauf mention contraire, on entend par "polymère cationique" (ou "polymère de type cationique") un polymère comportant des fonctions chimiques de type cationique. On peut aussi parler de polyélectrolyte cationique.

De préférence, le polymère cationique est lipophile ou liposoluble.

Selon un mode de réalisation, la phase grasse d'une émulsion selon l'invention, notamment la phase grasse dispersée, comprend au moins un polymère cationique.

Dans le cadre de la présente demande, et sauf mention contraire, par "fonction chimique de type cationique", on entend une fonction chimique B capable de capter un proton pour donner une fonction BH⁺. Selon les conditions du milieu dans lequel il se trouve, le polymère de type cationique comporte donc des fonctions chimiques sous forme B, ou bien sous forme BH⁺, son acide conjugué.

Comme exemple de fonctions chimiques de type cationique, on peut citer les fonctions amine primaire, secondaire et tertiaire, éventuellement présentes sous forme de cations ammoniums.

Comme exemple de polymère cationique, on peut citer tout polymère formé par la polymérisation de monomères dont au moins une partie porte des fonctions chimiques de type cationique, tel que des fonctions amine primaire, secondaire ou tertiaire.

De tels monomères sont par exemple l'aziridine, ou tout monomère éthyléniquement insaturé comportant au moins une fonction amine primaire, secondaire ou tertiaire.

Parmi les exemples de polymères cationiques appropriés à la mise en œuvre de l'invention, on peut citer l'amodiméthicone, dérivé d'un polymère silicone (polydiméthylsiloxane, aussi appelé diméthicone), modifié par des fonctions amine primaire et amine secondaire.

On peut également citer des dérivés de l'amodiméthicone, comme par exemple des copolymères de l'amodiméthicone, l'aminopropyl diméthicone, et plus généralement des polymères silicones linéaires ou ramifiés comportant des fonctions aminés.

On peut citer le copolymère de bis-isobutyl PEG-14/amodiméthicone, le Bis (C13-15 Alkoxy) PG-Amodimethicone, le Bis-Cetearyl Amodimethicone et le bis-hydroxy/méthoxy amodiméthicone.

On peut également citer les polymères de type polysaccharide comprenant des fonctions amine, tel que le chitosan ou les dérivés de gomme guar (chlorure d'hydroxypropyltrimonium guar).

On peut également citer les polymères de type polypeptide comprenant des fonctions amine, tel que la polylysine.

On peut également citer les polymères de type polyéthylèneimine comprenant des fonctions amine, tel que la polyéthylèneimine linéaire ou branchée.

Selon un mode de réalisation, les gouttes, et notamment l'écorce desdites gouttes, comprennent un polymère cationique qui est un polymère silicone modifié par une fonction amine primaire, secondaire ou tertiaire, tel que l'amodiméthicone.

Selon un mode de réalisation, les gouttes, et en particulier l'écorce desdites gouttes, comprennent de l'amodiméthicone.

Selon un mode de réalisation particulièrement préféré, le polymère cationique répond à la formule suivante : dans laquelle : 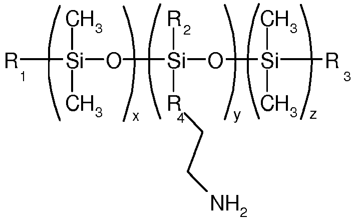
- R₁, R₂ et R₃, indépendamment les uns des autres, représentent OH ou CH₃ ;
- R₄ représente un groupe -CH₂- ou un groupe -X-NH- dans lequel X est un radical alkylène divalent en C3 ou C4 ;
- x est un nombre entier compris entre 10 et 5 000, de préférence entre 30 et 1 000, et mieux entre 80 et 300 ;
- y est un nombre entier compris entre 2 et 1 000, de préférence entre 4 et 100, et mieux entre 5 et 20 ; et
- z est un nombre entier compris entre 0 et 10, de préférence entre 0 et 1, et mieux est égal à 1.

Dans la formule susmentionnée, lorsque R₄ représente un groupe -X-NH-, X est relié à l'atome de silicium.

Dans la formule susmentionnée, R₁, R₂ et R₃ représentent de préférence CH₃.

Dans la formule susmentionnée, R₄ est de préférence un groupe -(CH₂)₃-NH-.

Selon l'invention, chaque goutte peut comprendre de 0,01% à 10%, de préférence de 0,05% à 5%, en poids de polymère(s) cationique(s), notamment d'amodiméthicone(s), par rapport au poids total de la phase grasse.

### Phase aqueuse

Outre le polymère anionique tel que défini ci-dessus, la phase aqueuse, notamment continue, des émulsions selon l'invention peut comprendre de l'eau.

De préférence, lorsque la phase aqueuse d'une émulsion selon l'invention est la phase continue, cette dernière se présente avantageusement sous forme d'un gel.

Outre l'eau distillée ou déionisée, une eau convenant à l'invention peut être aussi une eau de source naturelle ou une eau florale.

Selon un mode de réalisation, le pourcentage massique d'eau de la phase continue aqueuse est d'au moins 40%, et mieux au moins 50%, notamment compris entre 70% et 98%, préférentiellement compris entre 75% et 95%, par rapport à la masse totale de ladite phase continue.

Une émulsion selon l'invention peut comprendre au moins 20%, de préférence au moins 30%, en particulier au moins 40%, et mieux au moins 50% en poids d'eau par rapport au poids total de ladite émulsion.

De préférence, les émulsions selon l'invention comprennent au moins 75% en poids de phase aqueuse.

### Tampon

La phase aqueuse, notamment la phase aqueuse continue, d'une émulsion selon l'invention peut en outre comprendre au moins un tampon. Selon l'invention, le tampon utilisé présente un pKa compris de 4,0 à 9,0.

Dans le cadre de la présente invention, et sauf mention contraire, on entend par "tampon" une espèce chimique qui, en solution aqueuse, maintient le pH de la composition aqueuse dans laquelle il est solubilisé, et ce malgré l'addition de petites quantités d'un acide ou d'une base, ou malgré une dilution.

Selon un mode de réalisation, le tampon comporte une ou deux fonctions acide sulfonique, de préférence une seule.

De préférence, le pKa du tampon est compris entre 5,0 et 8,0, avantageusement entre 6,0 et 8,0.

Selon un mode de réalisation, le tampon est choisi dans le groupe constitué des tampons phosphate, de l'acide 2-(N-morpholino) éthane sulfonique (MES), du 2-amino-2-hydroxyméthyl-1,3-propanediol, de l'acide 2-(bis(2-5 hydroxyethyl)amino)acétique, de l'acide 4-(2-hydroxyéthyl)-1-pipérazine éthane sulfonique (HEPES), du citrate de sodium et de leurs mélanges.

Dans le cadre de la présente invention, et sauf mention contraire, on entend par « tampon phosphate » un tampon comprenant des ions dihydrogénophosphate et hydrogénophosphate.

Un tampon phosphate selon l'invention peut être préparé en dissolvant du phosphate monosodique ou monopotassique et du phosphate disodique ou dipotassique dans de l'eau.

A titre de tampon phosphate, on peut citer le PBS, qui désigne le tampon phosphate salin (pour « phosphate buffered saline »), préparé par dissolution de phosphate disodique (10 mM), de phosphate monopotassique (1,76 mM), de chlorure de sodium (137 mM) et de chlorure de potassium (2,7 mM) dans de l'eau. PBS possède un pKa de 7,2 et permet de tamponner une composition aqueuse dans une gamme de pH allant de 6,5 à 7,9.

A titre de tampon phosphate, on peut également citer le tampon préparé par dissolution de phosphate disodique (0,44% massique) et de phosphate monopotassique (2,74% massique) dans de l'eau. Un tel tampon possède un pKa de 5,8.

En particulier, le tampon est l'acide 4-(2-hydroxyéthyl)-1-pipérazine éthane sulfonique, notamment appelé HEPES (N° CAS 7365-45-9). HEPES possède un pKa de 7,5 et permet de tamponner une composition aqueuse dans une gamme de pH allant de 6,8 à 8,2.

Selon un mode de réalisation, la phase aqueuse, notamment la phase continue aqueuse, d'une émulsion selon l'invention peut comprendre de 0,1% à 10%, de préférence de 0,5% à 5%, en poids de tampon(s) par rapport au poids total de ladite phase aqueuse.

L'émulsion selon l'invention, notamment de type huile-dans-eau, peut comprendre de 0,1% à 10% en poids de tampon(s), de préférence de 0,5% à 5% en poids, par rapport au poids total de l'émulsion.

### Base

La phase continue aqueuse de l'émulsion selon l'invention peut en outre comprendre au moins une base. Elle peut comprendre une base unique ou un mélange de plusieurs bases différentes. La présence d'au moins une base dans ladite phase continue aqueuse contribue notamment à rehausser la viscosité de cette dernière.

Selon un mode de réalisation, la base présente dans la phase continue aqueuse est une base minérale, de préférence choisie dans le groupe constitué des hydroxydes des métaux alcalins et des hydroxydes des métaux alcalino-terreux.

De préférence, la base minérale est un hydroxyde de métaux alcalins, et notamment NaOH.

Selon un mode de réalisation, la base présente dans la phase continue aqueuse est une base organique. Parmi les bases organiques, on peut citer par exemple l'ammoniaque, la pyridine, la triéthanolamine, l'aminométhylpropanol, ou encore la triéthylamine.

Une émulsion de type huile-dans-eau selon l'invention peut comprendre de 0,01% à 10% en poids, de préférence de 0,01% à 5% en poids, et préférentiellement de 0,02% à 1% en poids de base, de préférence de base minérale, et notamment de NaOH, par rapport au poids total de ladite émulsion.

### Agent parfumant

Les émulsions selon l'invention comprennent en outre au moins un agent parfumant.

Comme il ressort des exemples ci-après, l'application au moyen d'un dispositif de conditionnement selon l'invention d'une émulsion selon l'invention comprenant au moins un agent parfumant, outre les avantages susmentionnés, offre :
- une meilleure rémanence de l'effet olfactif conféré par l'agent parfumant présent dans l'émulsion, et
- une puissance olfactive améliorée.

Sans vouloir être lié à une quelconque théorie, les performances olfactives améliorées observées avec le dispositif selon l'invention, peuvent se traduire par l'absence de dénaturation du parfum susceptible d'être réalisée avec des modes d'application « classiques », à savoir notamment de type spray ou avec une application au contact des doigts de l'utilisateur.

L'émulsion selon l'invention comprend au moins 2% en poids, en particulier au moins 3% en poids, de préférence de 3% à 35% en poids, en particulier de 5% à 20% en poids, et mieux de 5% à 15% en poids, et plus préférentiellement de 5% à 10% en poids, d'agent(s) parfumant(s) par rapport au poids total de l'émulsion.

Selon un mode de réalisation, la teneur d'agent(s) parfumant(s) est d'au moins 3%, de préférence d'au moins 5% en poids par rapport au poids total de ladite émulsion. En particulier, l'émulsion comprend de 5% à 10% en poids d'agent(s) parfumant(s) par rapport au poids total de ladite émulsion.

Selon l'invention, l'agent parfumant ou parfum peut être sous la forme d'un mélange. Ainsi, les gouttes selon l'invention peuvent comprendre un agent parfumant unique (ou parfum unique) ou un mélange de plusieurs agents parfumants (ou mélange de plusieurs parfums).

Parmi les agents parfumants, on peut notamment citer tout type de parfum ou de fragrance, ces termes étant utilisés ici de façon indifférente. Ces parfums ou fragrances sont bien connus de l'homme du métier et incluent notamment ceux mentionnés, par exemple, dans S. Arctander, Perfume and Flavor Chemicals (Montclair, N.J., 1969), S. Arctander, Perfume and Flavor Materials of Natural Origin (Elizabeth, N.J., 1960) et dans "Flavor and Fragrance Materials", 1991 (Allured Publishing Co. Wheaton, III. USA). Les parfums utilisés dans le cadre de la présente invention peuvent comprendre les produits naturels comme les extraits, les huiles essentielles, les absolus, les résinoïdes, les résines, les concrètes, etc... ainsi que les substances basiques de synthèse comme les hydrocarbures, les alcools, les aldéhydes, les cétones, les éthers, les acides, les esters, les acétals, les cétals, les nitriles, etc..., y compris les composés, saturés et insaturés, les composés aliphatiques, alicycliques et hétérocycliques.

Selon un mode de réalisation, l'agent parfumant comprend moins de 25% en poids d'alcool(s), linéaire(s) ou ramifié(s), saturé(s) ou comprenant éventuellement au moins une insaturation, par rapport au poids total dudit agent parfumant. Plus particulièrement, selon un mode de réalisation, l'agent parfumant comprend moins de 25% en poids d'alcool(s) terpénique(s) par rapport au poids total dudit agent parfumant.

Selon un mode de réalisation, l'agent parfumant comprend moins de 10%, voire moins de 5%, en poids d'aldéhyde(s), par rapport au poids total dudit agent parfumant.

Selon un mode de réalisation, l'agent parfumant comprend moins de 10%, voire moins de 7,5%, en poids de composé(s) avec un ClogP inférieur à 2,1, par rapport au poids total dudit agent parfumant.

Selon un mode de réalisation, l'agent parfumant comprend au moins 3%, voire au moins 4%, en poids d'alcool(s), linéaire(s) et/ou ramifié(s), et moins de 25%, voire moins de 20%, ou encore moins de 15%, en poids d'alcool(s), linéaire(s) et/ou ramifié(s), par rapport au poids total dudit agent parfumant.

Selon un mode de réalisation, l'agent parfumant comprend 4% en poids d'alcool(s), linéaire(s) et/ou ramifié(s), par rapport au poids total dudit agent parfumant.

Selon un mode de réalisation, l'agent parfumant comprend 13% en poids d'alcool(s), linéaire(s) et/ou ramifié(s) par rapport au poids total dudit agent parfumant, par rapport au poids total dudit agent parfumant.

Selon un mode de réalisation, l'agent parfumant ne comprend pas d'aldéhyde.

Selon un mode de réalisation, l'agent parfumant ne comprend pas de composé avec un ClogP inférieur à 2,1.

Selon un mode de réalisation, une goutte d'une émulsion comprenant au moins un agent parfumant selon l'invention comprend plus de 60%, voire plus de 70%, de préférence plus de 80%, et préférentiellement plus de 90%, en poids d'agent(s) parfumant(s) par rapport au poids total de ladite goutte.

De préférence, l'/les agent(s) parfumant est/sont présent(s) au niveau de la phase grasse ou de la phase dispersée d'une émulsion selon l'invention, et plus préférentiellement de la phase grasse dispersée.

Ainsi, selon l'invention, la phase dispersée, notamment la phase grasse dispersée, d'une émulsion selon l'invention comprend au moins 3% en poids, de préférence au moins 5% en poids d'agent(s) parfumant(s) par rapport au poids total de ladite phase dispersée.

### Polymère/copolymère réticulé

Selon un mode de réalisation, une émulsion selon l'invention, de préférence de type huile-dans-eau, et notamment la phase aqueuse de ladite émulsion, peut en outre comprendre au moins un polymère réticulé ou au moins un copolymère réticulé, ledit polymère réticulé ou copolymère réticulé comprenant au moins une unité dérivée de la polymérisation d'un des monomères suivants : acide acrylique ou méthacrylique, acrylate ou méthacrylate d'alkyle comprenant de 1 à 30 atomes de carbone, ou leurs sels (désigné ci-après « polymère/copolymère réticulé »), différent des polymères anioniques et cationiques décrits ci-dessus.

La présence d'un tel polymère/copolymère réticulé est particulièrement avantageuse dans le cas d'une émulsion selon l'invention comprenant au moins un agent parfumant, notamment présent dans la phase dispersée. En effet, la présence d'un tel polymère/copolymère réticulé contribue à stabiliser l'agent parfumant dans l'émulsion et ce, notamment sur une durée supérieure à un mois et à des températures comprises entre 5°C et 50°C, de préférence entre 10°C et 60°C.

Selon l'invention, le terme "unité dérivée de la polymérisation d'un monomère" signifie que le polymère ou copolymère est un polymère ou copolymère obtenu par polymérisation ou copolymère dudit monomère.

Selon un mode de réalisation, le polymère réticulé ou le copolymère réticulé est un polyacrylate réticulé.

Les copolymères et polymères réticulés de l'invention sont anioniques.

Selon un mode de réalisation, le copolymère est un copolymère d'acide carboxylique insaturé et de carboxylate insaturé d'alkyle en C₁₋₃₀, de préférence en C₁-C₄. Un tel copolymère comporte au moins un motif hydrophile de type acide carboxylique insaturé oléfinique et au moins un motif hydrophobe de type ester d'alkyle (C₁-C₃₀) d'acide carboxylique insaturé.

De préférence, ces copolymères sont choisis parmi ceux dont le motif hydrophile de type acide carboxylique insaturé oléfinique correspond au monomère de formule (I) suivante : 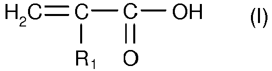
dans laquelle : R₁ désigne H ou CH₃ ou C₂H₅, c'est-à-dire des motifs acide acrylique, acide méthacrylique ou acide éthacrylique,
et dont le motif hydrophobe de type ester d'alkyle (C₁-C₃₀) d'acide carboxylique insaturé correspond au monomère de formule (II) suivante : 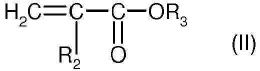
dans laquelle : R₂ désigne H ou CH₃ ou C₂H₅ (c'est-à-dire des motifs acrylates, méthacrylates ou éthacrylates) et de préférence H (motifs acrylates) ou CH₃ (motifs méthacrylates), R₃ désignant un radical alkyle en C₁-C₃₀, et de préférence en C₁-C₄.

Parmi ce type de copolymères, on utilisera plus particulièrement ceux formés à partir d'un mélange de monomères comprenant :
(i) essentiellement de l'acide acrylique,
(ii) un ester de formule (II) décrite ci-dessus et dans laquelle R₂ désigne H ou CH₃, R₃ désignant un radical alkyle ayant de 1 à 4 atomes de carbone, et
(iii) un agent réticulant, qui est un monomère insaturé polyéthylénique copolymérisable bien connu, comme le phtalate de diallyle, le triméthylolpropane tri(meth)acrylate, diallyl itaconate, diallyl fumarate, diallyl maléate, zinc (meth)acrylate, le (méth)acrylate d'allyle, le divinylbenzène, le diméthacrylate de (poly)éthylèneglycol, le méthylène-bis-acrylamide, et l'huile de ricin.

Selon un mode de réalisation, le polymère ou le copolymère est un polymère ou copolymère d'acide acrylique et/ou d'acide méthacrylique et/ou d'acrylate d'alkyle comprenant de 1 à 30 atomes de carbone, de préférence de 1 à 4 atomes de carbone, et/ou de méthacrylate d'alkyle comprenant de 1 à 30 atomes de carbone, de préférence de 1 à 4 atomes de carbone.

Selon un mode de réalisation, le copolymère réticulé est un copolymère réticulé d'acide méthacrylique et d'acrylate d'alkyle comprenant de 1 à 4 atomes de carbone, de préférence 2 atomes de carbone.

Dans le cadre de l'invention, et sauf mention contraire, on entend par « copolymère réticulé d'acide méthacrylique et d'acrylate d'alkyle comprenant de 1 à 4 atomes de carbones », un copolymère réticulé résultant de la polymérisation d'un monomère d'acide méthacrylique et d'un monomère d'acrylate d'alkyle comprenant de 1 à 4 atomes de carbones.

De préférence, dans ce copolymère, l'acide méthacrylique représente de 20% à 80% en poids, de préférence de 35% à 65% en poids du poids total du copolymère.

De préférence, dans ce copolymère, l'acrylate d'alkyle représente de 15% à 80% en poids, de préférence de 35% à 65% en poids du poids total du copolymère.

En particulier, l'acrylate d'alkyle est choisi parmi le méthacrylate d'alkyle, l'acrylate d'éthyle et l'acrylate de butyle.

Selon un mode de réalisation, le polymère réticulé ou le copolymère réticulé selon l'invention, présent dans la phase aqueuse continue, est choisi dans le groupe constitué des polymères ou copolymères suivants : Acrylates Copolymer, Acrylates crosspolymer-4, Acrylates crosspolymer-3, Polyacrylate-2 Crosspolymer et Polyacrylate-14 (noms INCI).

Parmi lesdits polymères ci-dessus, on préfère tout particulièrement selon la présente invention, les produits vendus par la société LUBRIZOL sous les dénominations commerciales Fixate Superhold (nom INCI = Polyacrylate-2 Crosspolymer), Fixate Freestyle Polymer (nom INCI = Acrylates crosspolymer-3), Carbopol^{®} Aqua SF1 (nom INCI = Acrylates copolymer) et Carbopol^{®} Aqua SF2 (nom INCI = Acrylates crosspolymer-4).

De préférence, le polymère réticulé ou le copolymère réticulé est choisi parmi le Carbopol^{®} Aqua SF1 (nom INCI = Acrylates copolymer) et/ou le Carbopol^{®} Aqua SF2 (nom INCI = Acrylates crosspolymer-4).

En particulier, il s'agit du Carbopol^{®} Aqua SF1 (nom INCI = Acrylates copolymer).

Selon un mode de réalisation, le copolymère réticulé est choisi parmi les copolymères réticulés d'acide acrylique ou méthacrylique et d'acrylates d'alkyle comprenant de 1 à 4 atomes de carbone.

Selon l'invention, la dispersion de l'invention peut comprendre de 0,1% à 10% en poids, de préférence de 0,5% à 8% en poids, et préférentiellement de 1% à 3% en poids de polymère(s) réticulé(s) ou copolymère(s) réticulé(s) par rapport au poids total de ladite dispersion.

### Agent(s) de texture

Une émulsion selon l'invention peut en outre comprendre un ou plusieurs agent(s) de texture, différents des polymères cationiques, des polymères anioniques et, lorsque présent, du polymère/copolymère réticulé décrits ci-dessus. Par agent de texture, on entend, au sens de l'invention, tout composé destiné à moduler la consistance et le toucher, à maintenir ou modifier la texture d'une émulsion selon l'invention.

Bien entendu, l'homme du métier veillera à choisir le(s) agent(s) de texture et/ou leur quantité en fonction de de la nature aqueuse ou grasse de la phase considérée et/ou de telle manière que (i) les propriétés avantageuses d'une émulsion selon l'invention et (ii) l'intégrité des gouttes formant ladite émulsion ne soient pas ou substantiellement pas altérées par l'adjonction envisagée. Ces ajustements relèvent des compétences de l'homme du métier.

Ainsi, dans une émulsion selon l'invention, la phase aqueuse peut comprendre au moins un agent de texture et/ou la phase grasse peut comprendre un agent de texture, différent(s) du polymère anionique, du polymère cationique et, lorsque présent, du polymère/copolymère réticulé.

Comme agents de texture hydrophiles, c'est-à-dire solubles ou dispersibles dans l'eau, et donc pouvant être présents dans la phase aqueuse d'une composition selon l'invention, on peut citer :
- les agents de texture naturels, notamment choisis parmi les extraits d'algues, les exsudats de plantes, les extraits de graines, les exsudats de microorganismes, et autres agents naturels,
- les agents de texture semi-synthétiques, notamment choisis parmi les dérivés de la cellulose et les amidons modifiés,
- les agents de texture synthétiques, notamment choisis parmi les homopolymères d'acide (méth)acrylique ou un de leurs esters, les copolymères d'acide (méth)acrylique ou un de leurs esters, les copolymères d'AMPS (2-acrylamido-2-méthylpropane sulfoniques acide), les polymères associatifs,
- les autres agents de texture, notamment choisis parmi les polyéthylèneglycols (commercialisé sous la dénomination Carbowax), les argiles, les silices telles que celles commercialisées sous les dénominations Aérosil ^{®} 90/130/150/200/300/380), la glycérine, et
- leurs mélanges.

Par « polymère associatif » au sens de la présente invention, on entend tout polymère amphiphile comportant dans sa structure au moins une chaîne grasse et au moins une portion hydrophile ; les polymères associatifs conformes à la présente invention peuvent être anioniques, cationiques, non-ioniques ou amphotères ; il s'agit notamment de ceux décrits dans FR 2 999 921. De préférence, il s'agit des polymères associatifs amphiphiles et anioniques et des polymères associatifs amphiphiles et non-ioniques tels que décrits ci-après.

Parmi les agents de texture naturels, on peut plus particulièrement citer les extraits d'algues figurés par l'agar-agar, les carraghénanes, les alginates, et leurs mélanges.

Parmi les agents de texture naturels, on peut plus particulièrement citer les exsudats de plantes figurés par la gomme adragante, la gomme de Karaya, la gomme de gatty, la gomme arabique, et leurs mélanges.

Parmi les agents de texture naturels, on peut plus particulièrement citer les extraits de graines figurés par la gomme de caroube, la gomme de guar, la gomme de tara, la gomme de konjac, les pectines, et leurs mélanges.

Parmi les agents de texture naturels, on peut plus particulièrement citer les exsudats de microorganismes figurés par la gomme de xanthane, la gomme de gellane, la pullulane, et leurs mélanges.

Parmi les agents de texture naturels, on peut encore citer d'autres agents naturels figurés notamment par la gélatine, le collagène, la kératine, les protéines végétales en particulier de blé et/ou de soja, les polymères de chitine ou de chitosane anioniques, cationiques, non-ioniques ou amphotères, l'acide hyaluronique ou un de ses sels, notamment le hyaluronate de sodium tel que celui commercialisé sous les dénominations HA Oligo, SC Hyaluronic Acid ou HyaCare, et leurs mélanges.

Parmi les agents de texture semi-synthétiques, les dérivés de la cellulose sont notamment figurés par la carboxyméthylcellulose (CMC) telle que celle commercialisée sous les dénominations Aqualon series ou Walocel sériés ; l'hydroxypropylcellulose (HPC) telle que celle commercialisée sous la dénomination Klucel HPC ; l'hydroxyéthylcellulose (HEC) telle que celle commercialisée sous les dénominations Cellosize series ou Natrosol 250 sériés ; l'hydroxyéthyl méthylcellulose telle que celle commercialisée sous la dénomination Walocel sériés ; l'hydroxypropyl méthylcellulose telle que celle commercialisée sous les dénominations Methocel E/F/J/K series de Dow Chemicals, VIVAPHARM CS 152 HV, Benecel E4M, E10M, K100M; la méthylcellulose telle que celle commercialisée sous la dénomination Methocel A sériés ; l'éthylcellulose telle que celle commercialisée sous la dénomination Ethocel sériés ; la cellulose microcristalline telle que celle commercialisée sous la dénomination Avicel PH sériés ; l'alkylhydroxyéthylcellulose telle que la cétylhydroxyéthylcellulose commercialisée sous la dénomination Polysurf 67), et leurs mélanges.

Parmi les agents de texture semi-synthétiques, les amidons modifiés sont des dérivés de l'amidon résultant de la modification de l'amidon natif par éthérification, estérification ou réticulation, tels que notamment le carboxyméthylamidon sodique tel que celui commercialisé sous les dénominations COVAGEL, VlVASTAR^{®} CS 352 SV ou VIVASTAR CS 302 SV ; l'hydroxypropylamidon tel que celui commercialisé sous les dénominations Zeina B860, Amaze NI, Amycol SQ, Penon PKW ; l'hydroxypropylamidon phosphate tel que celui commercialisé sous les dénominations Structure ZEA/style/XL ; et leurs mélanges.

Parmi les agents de texture synthétiques, les homopolymères d'acide (méth)acrylique ou un de leurs esters sont notamment figurés par les polyacrylates de sodium tels que ceux commercialisés sous les dénominations Cosmedia SP, Covacryl MV60/MV40, Cosmedia SPL ou Luvigel EM ; les polymères d'acide (méth)acryliques réticulés (ou carbomères), différents des carbomères définis comme polymère anionique ci-dessus, tels que ceux commercialisés sous les dénominations Carbopol 900 series, Carbopol 2984/ 5984, Carbopol Ultrez 10/30, en particulier le Carbopole Ultrez 21, Tego Carbomer 134/ 140 / 141, Aqupec HV-505, HV-505HC, HV-504, HV-501, HV-505E, HV-504E, HV-501E, HV-505ED, Ashland 941 carbomer, ou Ashland 981 carbomer ; et leurs mélanges.

Parmi les agents de texture synthétiques, les copolymères d'acide (méth)acrylique ou un de leurs esters sont notamment figurés par l'acrylate de glycéryle/copolymère d'acide acrylique tels que celui commercialisé sous les dénominations Lubrajel series, Lubrasil series ou Norgel ; les copolymères acrylates tels que ceux commercialisés sous les dénominations ou Carbopol Aqua SF-1 OS Polymer (nom INCI = Acrylates copolymer); les sodium acrylates crosspolymer-2 tel que celui commercialisé sous la dénomination Aquakeep 10 SH NF ; les acrylates/C₁₀-C₃₀ alkyl acrylate crosspolymers tels que ceux commercialisés sous les dénominations Carbopol 1342/1382, Carbopol ETD 2020, Pemulen TR-1/TR-2, Carbopol Ultrez 20/21,Tego Carbomer 341 ER, Tego Carbomer 750 HD, Tego Carbomer 841 SER, Aqupec HV-501ER, HV-701EDR, HV-501EM, SER W-150C ou SER W-300C ; les sodium acrylates/beheneth-25 méthacrylate crosspolymer tels que celui commercialisé sous la dénomination Novemer EC-2 ; les acrylates/acrylamide copolymers tel que celui commercialisé sous la dénomination Novemer EC-1 par Lubrizol ; les acrylamide/sodium acrylate copolymers tel que celui commercialisé sous la dénomination Aquagel 55 ; les acrylic Acid/VP crosspolymers tel que celui commercialisé sous la dénomination Ultrathix P-100; et leurs mélanges.

Parmi les agents de texture synthétiques, les copolymères d'AMPS sont notamment figurés par les copolymères AMPS NH4/Vinylpyrrolidone tel que celui commercialisé sous la dénomination Aristoflex AVC (INCI: Ammonium Acryloyldimethyltaurate/VP Copolymer) ; les copolymère AMPS NH4/Beheneth-25 méthacrylate tel que celui commercialisé sous la dénomination Aristoflex HMB (INCI: Ammonium Acryloyldimethyltaurate/Beheneth-25 methacrylate Crosspolymer) ; les copolymères AMPS Na/Vinylpyrrolidone tel que celui commercialisé sous la dénomination Aristoflex AVS (INCI: Sodium Acryloyldimethyl taurate/VP Copolymer) ; les copolymères AMPS NH4/2-Carboxyéthylacrylate tel que celui commercialisé sous la dénomination Aristoflex TAC (INCI: Ammonium Acryloyldimethyltaurate/carboxyethyl crosspolymer) ; les copolymères AMPS Na/Acrylic acid/Sodium Acrylate/Diméthyl acrylamide tel que celui commercialisé sous la dénomination Simulgel SMS88 (INCI: Sodium Acrylate/Acryloyldimethyltaurate/Dimethylacrylamide Crosspolymer & Isohexadecane & Polysorbate 60); les copolymères AMPS Na/Sodium Acrylate tels que ceux commercialisés sous les dénominations Simulgel EG (INCI: Sodium Acrylate/Sodium Acryloyldimethyl Taurate Copolymer & Isohexadecane & Polysorbate 80) ou Simulgel EPG (INCI: Sodium Acrylate/Sodium Acryloyldimethyl Taurate Copolymer & Polyisobutene & Caprylyl/Capryl Glucoside) ; les copolymères AMPS Na/ Acrylamide tels que ceux commercialisés sous les dénominations Simulgel 600 (INCI: Acrylamide/Sodium Acryloyldimethyltaurate/lsohexadecane/Polysorbate-80) ou Sepigel 305 (INCI: Polyacrylamide/C13-C14 Isoparaffin/Laureth-7) ; les Methyl Methacrylate Crosspolymer tels que ceux commercialisés sous les dénominations SEPIMAT^{™} SB par la société SEPPIC ; les copolymères AMPS Na/hydroxyéthyl acrylate tels que ceux commercialisés sous les dénominations Simulgel NS (INCI: hydroxyéthyl acrylate/sodium acryloyldimethyltaurate copolymer & squalane & polysorbate-60), Simulgel INS 100 (INCI: Hydroxyethyl Acrylate/Sodium Acryloyldimethyl Taurate Copolymer & Isohexadecane & Polysorbate 60), Simulgel FL (INCI: Hydroxyethyl Acrylate/Sodium Acryloyldimethyl Taurate Copolymer & Isohexadecane & Polysorbate 60), Sepinov WEO ou Sepinov EMT 10 (INCI: hydroxyéthyl acrylate/sodium acryloyldimethyltaurate copolymer) ; les acryloyl Dimethyltaurate/Sodium Acrylate/Dimethylacrylamide crosspolymers tel que celui commercialisé sous la dénomination Sepinov P88 (INCI: Sodium Acrylate/Acryloyldimethyltaurate/Dimethylacrylamide Crosspolymer) ; et leurs mélanges.

Parmi les agents de texture synthétiques, on peut en outre citer le PVP tel que celui commercialisé sous la dénomination FlexiThix polymer ou le Nylon-6 tel que celui commercialisé sous la dénomination Orgasol^{®} 1002 D NAT COS.

Parmi les agents de texture synthétiques, les polymères associatifs amphiphiles et anioniques sont notamment figurés par les acrylates/Steareth-20 Methacrylate Copolymer tel que celui commercialisé sous la dénomination Aculyn 22 ; les acrylates/Beheneth-25 Methacrylate Copolymer tel que celui commercialisé sous la dénomination Aculyn 28; les C₃₀-₃₈ Olefin/Isopropyl Maleate/MA Copolymer tel que celui commercialisé sous la dénomination Performa V 1608 ; les Acrylates/Steareth-20 Methacrylate Crosspolymer tel que celui commercialisé sous la dénomination Aculyn 88; le Polyacrylate Crosspolymer-6 tel que celui commercialisé sous la dénomination Sepimax Zen; les Acrylates/C₁₀-C₃₀ Alkyl Acrylate Crosspolymers tels que ceux susmentionnés; et leurs mélanges.

Parmi les agents de texture synthétiques, les polymères associatifs amphiphiles et non-ioniques sont notamment figurés par le PEG-150 distearate tel que celui commercialisé sous la dénomination Emanon 3299V; les PEG-150/Decyl Alcohol/SMDI Copolymer tel que celui commercialisé sous la dénomination Aculyn 44; les PEG-150/stearyl alcohol/SMDI copolymer tel que celui commercialisé sous la dénomination Aculyn 46; les acrylates/ceteth-20 itaconate copolymer tel que celui commercialisé sous la dénomination Structure 3001 par AkzoNobel Personal Care; les polyurethane polyethers tels que ceux commercialisés sous les dénominations Rheolate FX 1100, Rheolate 205, Rheolate 208 / 204 / 212, Elfacos T1212, Acrysol RM 184 / RM 2020, Adeka Nol GT-700 / GT-730 ; le polyurethane-39 tel que celui commercialisé sous la dénomination Luvigel Star ; les cetyl hydroxyethylcellulose tels que ceux commercialisés sous les dénominations Natrosol^{™} Plus ou PolySurf^{™} 67 ; et leurs mélanges.

Comme agents de texture de la phase aqueuse, on peut encore citer les argiles, notamment figurées par la bentonite telle que celle commercialisée sous les dénominations Veegum, Veegum HS ou Vanatural ; la montmorillonite, l'hectorite telle que celle commercialisée sous les dénominations Bentone series ou Hectone sériés ; la kaolinite, et leurs mélanges.

De préférence, la phase aqueuse, notamment continue, peut comprendre à titre d'agent de texture au moins un copolymère réticulé tel que décrit ci-dessous, et en particulier le Carbopol^{®} Aqua SF1 (nom INCI = Acrylates copolymer).

Les agents de texture utilisables selon l'invention peuvent être des agents de texture lipophiles organiques ou minéraux, polymériques ou moléculaires.

Comme agent de texture lipophile minéral, on peut citer les argiles éventuellement modifiées comme les hectorites modifiées par un chlorure d'ammonium en C₁₀ à C₂₂, comme l'hectorite modifiée par du chlorure de di-stéaryl di-méthyl ammonium telle que, par exemple, celle commercialisée sous la dénomination de Bentone 38V^{®} par la société ELEMENTIS.

On peut également citer la silice pyrogénée éventuellement traitée hydrophobe en surface dont la taille des particules est inférieure à 1 µm. Il est en effet possible de modifier chimiquement la surface de la silice, par réaction chimique générant une diminution du nombre de groupes silanol présents à la surface de la silice. On peut notamment substituer des groupes silanol par des groupements hydrophobes : on obtient alors une silice hydrophobe.

Les groupements hydrophobes peuvent être :
- des groupements triméthylsiloxyle, qui sont notamment obtenus par traitement de silice pyrogénée en présence de l'hexaméthyldisilazane. Des silices ainsi traitées sont dénommées « Silica silylate » selon le CTFA (8ème édition, 2000). Elles sont par exemple commercialisées sous les références Aerosil R812^{®} par la société DEGUSSA, CAB-O-SIL TS-530^{®} par la société CABOT ; ou
- des groupements diméthylsilyloxyle ou polydiméthylsiloxane, qui sont notamment obtenus par traitement de silice pyrogénée en présence de polydiméthylsiloxane ou du diméthyldichlorosilane. Des silices ainsi traitées sont dénommées « Silica diméthyl silylate » selon le CTFA (8ème édition, 2000). Elles sont par exemple commercialisées sous les références Aerosil R972^{®}, et Aerosil R974^{®} par la société DEGUSSA, CAB-O-SIL TS-610^{®} et CAB-O-SIL TS-720^{®} par la société CABOT.

La silice pyrogénée hydrophobe présente en particulier une taille de particules pouvant être nanométrique à micrométrique, par exemple allant d'environ de 5 à 200 nm.

Les agents de texture lipophiles organiques polymériques sont par exemple les organopolysiloxanes élastomériques partiellement ou totalement réticulés, de structure tridimensionnelle, comme ceux commercialisés sous les dénominations de KSG6^{®}, KSG16^{®} et de KSG18^{®} par la société SHIN-ETSU, de Trefil E-505C^{®} et Trefil E-506C^{®} par la société DOW-CORNING, de Gransil SR-CYC^{®}, SR DMF10^{®}, SR-DC556^{®}, SR 5CYC gel^{®}, SR DMF 10 gel^{®} et de SR DC 556 gel^{®} par la société GRANT INDUSTRIES, de SF 1204^{®} et de JK 113^{®} par la société GENERAL ELECTRIC ; l'éthylcellulose comme celle vendue sous la dénomination Ethocel^{®} par la société DOW CHEMICAL ; les galactommananes comportant de un à six, et en particulier de deux à quatre, groupes hydroxyle par ose, substitués par une chaîne alkyle saturée ou non, comme la gomme de guar alkylée par des chaînes alkyle en C₁ à C₆, et en particulier en C₁ à C₃ et leurs mélanges. Les copolymères séquencés de type « dibloc », « tribloc » ou « radial » du type polystyrène/polyisoprène, polystyrène/polybutadiène tels que ceux commercialisés sous la dénomination Luvitol HSB^{®} par la société BASF, du type polystyrène/copoly(éthylènepropylène) tels que ceux commercialisés sous la dénomination de Kraton^{®} par la société SHELL CHEMICAL CO ou encore du type polystyrène/copoly(éthylène-butylène), les mélanges de copolymères tribloc et radial (en étoile) dans l'isododécane tels que ceux commercialisé par la société PENRECO sous la dénomination Versagel^{®} comme par exemple le mélange de copolymère tribloc butylène/éthylène/styrène et de copolymère étoile éthylène/propylène/styrène dans l'isododécane (Versagel M 5960).

Comme agent de texture lipophile, on peut encore citer les polymères de masse moléculaire moyenne en poids inférieure à 100 000, comportant a) un squelette polymérique ayant des motifs de répétition hydrocarbonés pourvus d'au moins un hétéroatome, et éventuellement b) au moins une chaîne grasse pendante et/ou au moins une chaîne grasse terminale éventuellement fonctionnalisées, ayant de 6 à 120 atomes de carbone et étant liées à ces motifs hydrocarbonés, telles que décrites dans les demandes WO 02/056847, WO 02/47619, en particulier les résines de polyamides (notamment comprenant des groupes alkyles ayant de 12 à 22 atomes de carbone) telles que celles décrites dans US 5783657.

A titre d'exemple de résine de polyamide pouvant être mise en œuvre selon la présente invention, on peut citer UNICLEAR 100 VG^{®} commercialisé par la société ARIZONA CHEMICAL.

Parmi les agents de texture lipophiles pouvant être utilisés dans les émulsions selon l'invention, on peut encore citer les esters de dextrine et d'acide gras, tels que les palmitates de dextrine, notamment tels que ceux commercialisés sous les dénominations Rheopearl TL^{®} ou Rheopearl KL^{®} par la société CHIBA FLOUR.

On peut également utiliser les polyamides siliconés du type polyorganosiloxane tels que ceux décrits dans US 5 874 069, US 5 919 441, US 6 051 216 et US 5 981 680.

Ces polymères siliconés peuvent appartenir aux deux familles suivantes :
- des polyorganosiloxanes comportant au moins deux groupes capables d'établir des interactions hydrogène, ces deux groupes étant situés dans la chaîne du polymère, et/ou
- des polyorganosiloxanes comportant au moins deux groupes capables d'établir des interactions hydrogène, ces deux groupes étant situés sur des greffons ou ramifications.

### Composé(s) additionnel(s)

Les émulsions de l'invention peuvent en outre comprendre des poudres, des paillettes, des colorants, des conservateurs, des humectants, des stabilisateurs, des chélateurs, des émollients etc... ou tout additif cosmétique usuel, et leurs mélanges.

Les émulsions selon l'invention peuvent encore en outre comprendre au moins un actif, de préférence choisi parmi les agents hydratants, les agents cicatrisants, les agents dépigmentants, les filtres UV, les agents desquamants, les agents antioxydants, les actifs stimulant la synthèse des macromoléculaires dermiques et/ou épidermiques, les agents dermodécontractants, les agents anti-transpirants, les agents apaisants, les agents anti-âge et leurs mélanges.

Bien entendu, l'homme du métier veillera à choisir le(s) composé(s) additionnel(s) et/ou leur quantité en fonction de de la nature aqueuse ou grasse de la phase considérée et/ou de telle manière que (i) les propriétés avantageuses d'une émulsion selon l'invention et (ii) l'intégrité des gouttes formant ladite émulsion ne soient pas ou substantiellement pas altérées par l'adjonction envisagée. Ces ajustements relèvent des compétences de l'homme du métier.

En particulier, et comme il ressort de l'exemple 3 ci-après, la mise en œuvre d'un antioxydant permet de réduire, voire prévenir, efficacement le phénomène de jaunissement d'une émulsion selon l'invention présente dans un dispositif de conditionnement selon l'invention qui, pour des raisons évidentes, n'est pas souhaitable. Cela conduit ainsi à renforcer encore la stabilité d'une émulsion selon l'invention.

Une émulsion selon l'invention comprend ainsi avantageusement une quantité efficace en antioxydant(s), en particulier une quantité efficace pour réduire, voire prévenir, le phénomène de jaunissement d'une émulsion selon l'invention présente dans un dispositif de conditionnement selon l'invention.

Avantageusement, une émulsion selon l'invention comprend une teneur en antioxydant(s) :
- supérieure à 0 % en poids par rapport au poids total de ladite émulsion, et
- inférieure ou égal à 0,04% en poids par rapport au poids total de ladite émulsion, et en particulier comprise entre 0,002% et 0,03%, de préférence entre 0,004% et 0,02%, en poids par rapport au poids total de ladite émulsion.

Avantageusement, une émulsion selon l'invention comprend un rapport pondéral « antioxydant(s) / agent(s) parfumant(s) » compris entre 2,8.10⁻⁵ et 0,02, de préférence entre 5,7.10⁻⁵ et 0,015, en particulier entre 1.10⁻⁴ et 0,01, et mieux entre 2.10⁻⁴ et 0,008.

Selon un mode de réalisation, les émulsions de l'invention, en particulier dans le cas d'une émulsion de type huile-dans-eau, comprennent de la glycérine. De préférence, les émulsions de l'invention comprennent au moins 5% en poids de glycérine par rapport au poids total desdites émulsions. En effet, au-delà de la texture, les émulsions selon l'invention apportent un autre avantage par rapport aux émulsions « classiques » car elles permettent d'utiliser de la glycérine, qui plus est dans des teneurs élevées.

Elles peuvent en particulier comprendre de la glycérine en une teneur supérieure ou égale à 10%, supérieure ou égale à 20%, supérieure ou égale à 30%, supérieure ou égale à 40%, voire jusqu'à 50%, en poids, par rapport au poids total de ladite émulsion.

Pour des raisons évidentes, la glycérine est présente au niveau de la phase aqueuse d'une émulsion selon l'invention.

Dans le cas d'émulsions comprenant une teneur élevée en glycérine, les inventeurs ont pu avantageusement observer une absence d'effet collant de l'émulsion lors de l'application de ladite émulsion au moyen du dispositif selon l'invention.

### Procédé de préparation

Les émulsions selon l'invention présentent l'avantage de pouvoir être préparées selon un procédé simple « non-microfluidique », à savoir par simple émulsification.

Comme dans une émulsion classique, une solution (ou phase) aqueuse et une solution (ou phase) grasse (ou huileuse) sont préparées séparément. C'est l'ajout sous agitation de la phase « grasse » dans la phase aqueuse qui crée une émulsion directe.

Les phases aqueuse et grasse sont donc préparées préalablement à leurs mises en contact pour former une émulsion selon l'invention. Lors de la préparation de la phase aqueuse et/ou de la phase grasse, la présence de certaines matières premières peut nécessiter des ajustements au niveau du procédé de préparation de ladite phase aqueuse et/ou de ladite phase grasse.

En particulier, une étape de chauffage (entre 40°C et 150°C, notamment entre 50°C et 90°C) peut être nécessaire dans la préparation de la phase grasse afin de solubiliser le(s) corps gras solide(s) à température et pression ambiante et/ou l'/les antioxydant(s) susceptible(s) d'y être incorporé(s).

Egalement, l'ajout du/des parfum(s) dans la phase grasse est avantageusement réalisé à température ambiante pour prévenir toute dégradation de sa note olfactive.

Par ailleurs, la présence, dans la phase grasse, de corps gras solide(s) à température et pression ambiante, telle qu'envisagée précédemment, peut nécessiter des ajustements au niveau du procédé de préparation d'une émulsion selon l'invention. En particulier, le procédé de préparation d'une telle émulsion selon l'invention peut comprendre une étape de chauffage (entre 40°C et 150°C, notamment entre 50°C et 90°C) de la phase grasse avant mélange/mise en contact de ladite phase grasse avec la phase aqueuse, voire le maintien de ce chauffage lors du mélange de la phase grasse avec la phase aqueuse et ce, avantageusement, jusqu'à obtention de l'émulsion souhaitée.

Ces ajustements relèvent des compétences générales de l'homme du métier.

Dans le cas d'une émulsion de type huile-dans-eau, les solutions (ou fluides) utilisés pour constituer la phase aqueuse continue et la phase grasse dispersée sont respectivement désignés Fluide Externe (FE) et Fluide Interne (FI).

Au vu de ce qui précède, le fluide FI comprend au moins un premier polymère précurseur du coacervat, notamment un polymère cationique, et en particulier l'amodiméthicone et en outre, de façon optionnelle, au moins une huile, au moins un corps gras solide à température et pression ambiante notamment tels que définis précédemment, au moins un agent parfumant et/ou au moins un composé additionnel susmentionné.

Le fluide FE comprend au moins de l'eau et au moins un deuxième polymère précurseur du coacervat, différent du premier polymère précurseur du coacervat, notamment un polymère anionique, et en particulier le carbomère, et en outre, de façon optionnelle, au moins un polymère/copolymère réticulé, au moins un agent parfumant et/ou au moins un composé additionnel tel(s) que susmentionné(s), voire une base, des conservateurs et/ou d'autres produits solubles dans l'eau tels que la glycérine.

Selon un mode de réalisation, le procédé de préparation d'une émulsion selon l'invention de type huile-dans-eau comprend une étape de formation des gouttes comprenant :
- la mise en contact d'un fluide FE et d'un fluide FI tels que définis ci-dessus ; et
- la formation des gouttes de phase grasse, constituée du fluide FI, dispersée dans une phase aqueuse continue constituée de fluide FE, lesdites gouttes comprenant une écorce isolant le cœur des gouttes de la phase grasse de la dispersion.

Selon un mode de réalisation, l'étape de formation des gouttes peut en outre comprendre une étape d'injection d'une solution d'augmentation de la viscosité de la phase aqueuse continue du fluide FE. De préférence, la solution d'augmentation de la viscosité est aqueuse. Cette solution d'augmentation de la viscosité est typiquement injectée dans le fluide externe aqueux FE après formation de la dispersion selon l'invention, et donc après formation des gouttes.

Selon un mode de réalisation, la solution d'augmentation de la viscosité comprend une base, notamment un hydroxyde d'alcalin, tel que l'hydroxyde de sodium.

Selon un mode de réalisation, en particulier lorsque le FI comprend au moins un corps gras solide à température et pression ambiante et/ou au moins un antioxydant tels que décrits précédemment, le procédé de préparation d'une émulsion selon l'invention peut en outre comprendre une étape de chauffage du fluide FI, à une température comprise de 40°C à 150°C, de préférence de 50°C à 90°C, préalablement à l'étape susmentionnée de formation des gouttes, et donc avant mélange/mise en contact de ladite phase grasse avec la phase aqueuse continue. Avantageusement, le procédé de préparation d'une émulsion selon l'invention comprenant un tel fluide FI est tel que les étapes de mise en contact d'un fluide FE et d'un fluide FI et de formation des gouttes de phase grasse, constituée du fluide FI, dispersée dans une phase aqueuse continue constituée de fluide FE, sont réalisées à une température comprise de 40°C à 150°C, de préférence de 50°C à 90°C. En d'autres termes, un tel procédé comprend le maintien du chauffage susmentionné lors du mélange de la phase grasse avec la phase aqueuse et ce, avantageusement, jusqu'à obtention de l'émulsion souhaitée.

Selon un mode de réalisation, le procédé de préparation peut en outre comprendre, entre l'étape de chauffage et l'étape de formation des gouttes, une étape consistant à abaisser la température du fluide FI, le cas échéant jusqu'à température ambiante.

### Utilisations

De manière préférée, l'émulsion selon l'invention est directement utilisable, à l'issue du procédé de préparation précité, à titre de composition, notamment cosmétique.

Une émulsion selon l'invention peut également se présenter sous la forme d'une composition, notamment cosmétique, et plus particulièrement une composition parfumante.

Une telle composition, notamment cosmétique, comprend au moins une émulsion selon l'invention en association avec un milieu physiologiquement acceptable.

Par "milieu physiologiquement acceptable", on entend désigner un milieu convenant particulièrement à l'application d'une composition de l'invention sur les matières kératiniques.

Selon un mode de réalisation, la composition est une composition cosmétique.

Par « matières kératiniques », on entend désigner notamment la peau, les lèvres, les ongles, les cils ou les sourcils, de préférence la peau, et tout particulièrement la peau du cou.

Le milieu physiologiquement acceptable est généralement adapté à la nature du support sur lequel doit être appliquée la composition, ainsi qu'à l'aspect sous lequel la composition doit être conditionnée.

Selon un mode de réalisation, le milieu physiologiquement acceptable est figuré directement par la phase continue aqueuse telle que décrite ci-dessus.

Selon un mode de réalisation, les émulsions ou compositions cosmétiques selon l'invention sont utilisées pour le maquillage et/ou le soin de matières kératiniques et/ou pour parfumer une matière kératinique, notamment de la peau.

De préférence, une composition selon l'invention ne comprend pas de tensio-actif.

Les compositions cosmétiques selon l'invention peuvent être des produits de parfum (ou produit parfumant), de soin, de protection solaire, de nettoyage (démaquillage), d'hygiène ou de maquillage de matières kératiniques, notamment de la peau.

Selon un mode de réalisation, les émulsions ou compositions de l'invention sont sous la forme d'un fond de teint, d'un démaquillant, d'un soin du visage et/ou du corps et/ou du cheveu, d'un soin anti-âge, d'un protecteur solaire, d'un soin peau grasse, d'un soin whitening, d'un soin hydratant, d'une BB cream, crème teintée ou fond de teint, d'un nettoyant visage et/ou corps, d'un gel douche ou d'un shampoing.

Selon un mode de réalisation, les émulsions ou compositions de l'invention ne sont pas des crèmes teintées ou des fonds de teint.

Une composition de soin selon l'invention peut être en particulier une composition solaire, une crème de soin, un sérum ou un déodorant.

De préférence, une composition selon l'invention, lorsqu'elle comprend au moins un agent parfumant, comme indiqué ci-dessus, est une composition parfumante (ou parfum). En d'autres termes, une telle composition selon l'invention est un parfum qui se distingue des parfums « classiques » par sa galénique et son mode d'application.

Selon un mode de réalisation où l'émulsion selon l'invention comprend au moins un agent parfumant et de la glycérine, qui plus est dans une teneur supérieure ou égale à 5% en poids par rapport au poids total de ladite émulsion, voire une composition comprenant ladite émulsion, ladite émulsion ou composition est particulièrement avantageuse en ce qu'elle peut être utilisée en tant que composition parfumante et de soin d'une matière kératinique, en particulier de la peau. Ce mode de réalisation est particulièrement intéressant du fait qu'il n'était pas évident que le caractère hydratant d'une telle composition n'altère pas l'effet olfactif recherché, et inversement.

Les émulsions ou compositions selon l'invention peuvent être sous diverses formes, notamment sous forme de crème, de baume, de lotion, de sérum, de gel, de gel-crème ou encore de brume.

La présente invention concerne également un procédé cosmétique non thérapeutique de maquillage et/ou de soin d'une matière kératinique et/ou pour améliorer la rétention de parfum sur une matière kératinique, comprenant les étapes suivantes :
i) mettre en contact le moyen d'application avec une émulsion du dispositif tel que décrit précédemment; et
ii) appliquer l'émulsion, présente sur le moyen d'application, sur une matière kératinique, de préférence sur la peau.

Selon un mode de réalisation, la mise en contact de l'applicateur avec l'émulsion est réalisée par pression de l'applicateur, en particulier par l'utilisateur, sur l'élément présentant au moins un orifice du réceptacle.

En particulier, lorsque l'élément est une éponge, l'émulsion est transférée de l'éponge à l'applicateur.

En particulier, lorsque l'élément est une grille ou un tamis, l'émulsion est transférée par le biais des trous (grille) ou des mailles (tamis) à l'applicateur.

Dans toute la description, y compris les revendications, l'expression « comprenant un » doit être comprise comme étant synonyme de « comprenant au moins un », sauf si le contraire est spécifié.

Les expressions « compris entre ... et ... », « compris de ... à ... » et « allant de ... à ... » doivent se comprendre bornes incluses, sauf si le contraire est spécifié.

Les quantités des ingrédients figurant dans les exemples sont exprimées en pourcentage en poids par rapport au poids total de la composition, sauf indication contraire.

### EXEMPLES

### Exemple 1 : Préparation d'une « Cushion cream » avec une composition parfumante de type émulsion huile-dans-eau

La composition de l'exemple 1 est constituée des ingrédients suivants :

| **Nom** | **Nom INCI** | **% w/w** |
|---|---|---|
| Eau osmosée | Aqua | 82,48% |
| Parfum | Fragrance | 10% |
| DUB ININ Grade A | Isononyl Isononanoate | 1 % |
| CAS3131 PILOT | Amodimethicone | 0,02% |
| Carbopol 981 | Carbomer | 0,18% |
| Crosspolymère Carbopol Aqua SF2 | Acrylates crosspolymer-4 | 2,70% |
| Microcare^{®} PE | Phénoxyéthanol | 0,72% |
| Microcare^{®} emollient PTG | Pentylène glycol | 1,80% |
| Hepes | Hydroxyethylpiperazine Ethane Sulfonic Acid Hydro | 0,90% |
| Edeta^{®} BD | Disodium EDTA | 0,01% |
| NaOH | Sodium Hydroxyde | 0,19% |
| Total | | 100,00% |

La composition de l'exemple 1 est préparée selon le protocole suivant :

### Préparation de la phase aqueuse :

On mélange la moitié d'eau finale et les conservateurs. On agite à l'aide d'une pale type défloculeuse pendant 15 minutes à 300 tpm.

On arrête l'agitation pour incorporer le carbomère, on attend 1 heure l'hydratation de celui-ci pour ensuite agiter à l'aide d'une défloculeuse rapidement mais sans incorporer de bulles d'air pendant 2 heures.

On ajoute le crosspolymère et on agite jusqu'à dispersion complète.

On prépare séparément une solution avec l'Hepes, la soude et le reste d'eau qui est ensuite ajoutée lentement à la solution précédente, toujours sous agitation.

### Préparation de la phase grasse (phase parfumante) :

Séparément, on prépare la phase parfumante en mélangeant l'isononyl isononanoate, l'amodiméthicone et le parfum.

### Préparation de l'émulsion :

La phase parfumante est ensuite incorporée lentement à la phase aqueuse sous agitation modérée jusqu'à dispersion totale.

En variante, la solution avec l'Hepes, la soude et le reste d'eau peut être ajoutée après dispersion de la phase parfumante dans la solution de carbomère.

Cette composition est ensuite placée dans un dispositif de conditionnement selon l'invention (i.e. de type « cushion cream ») ; dans le réceptacle dudit dispositif, la composition est imbibée/imprégnée au sein d'une éponge alvéolée (i.e. l'élément présentant au moins un orifice).

### Protocole d'application sur une matière kératinique

A l'aide d'une houppette (i.e. le moyen d'application), l'utilisateur exerce une pression sur l'éponge alvéolée comprenant la composition ci-dessus et peut ainsi prélever une quantité désirée de la composition, sans contact entre la main de l'utilisateur et ladite composition.

Egalement, l'utilisateur peut ajuster aisément la quantité de composition prélevée en exerçant une pression plus ou moins forte sur l'éponge. En d'autres termes, la houppette permet de capter la juste dose de la composition.

Ensuite, l'utilisateur met en contact la houppette revêtue au moins partiellement de la composition avec la peau du cou.

### Résultats

Préliminairement, eu égard à la finalité cosmétique considérée, à savoir parfumer, l'utilisateur est surpris du mode de conditionnement et de la galénique de la composition.

Ensuite, lors de l'application sur la peau de la composition ci-dessus à l'aide du dispositif selon l'invention, l'utilisateur constate les avantages suivants :
- une sensation de fraicheur sur la peau,
- la fourniture d'un produit léger,
- une application facile : la houppette qui sert à appliquer le produit est douce, agréable et facile à utiliser,
- la composition est multi-fonctions, voire sur-mesure : la houppette permet de capter la juste dose de la composition si bien que l'application de cette dernière permet de jouer sur les degrés de couvrance et/ou de charge de parfum, de la plus subtile à la plus intense,
- l'éponge est rechargeable, ce qui est écologique et permet de moduler la teinte selon la saison,
- aucun contact entre les doigts et la composition, et
- produit « nomade » car petit et facile à transporter.

### Exemple 2 : essai comparatif

Un comparatif est réalisé à partir de la composition décrite en exemple 1 ci-dessus, au niveau du mode d'application.

Chaque utilisateur considéré pour ce comparatif effectue une application sur la peau de la composition ci-dessus via deux modes d'application différents, à savoir :
- le premier mode d'application, sur la partie gauche du cou, correspond à une application « classique » de type spray, et
- le second mode d'application, sur la partie droite du cou, correspond à une application à l'aide du dispositif selon l'invention.

Les impressions des utilisateurs, notamment en termes de rémanence du parfum et de l'intensité de ce dernier, sont ensuite recueillies.

### Résultats

Outre les avantages susmentionnés, la composition appliquée sur la peau à l'aide du dispositif selon l'invention offre :
- une meilleure rémanence de l'effet olfactif conféré par l'agent parfumant présent dans la composition, et
- une puissance olfactive améliorée.

### Exemple 3 : essai comparatif sur le jaunissement de compositions parfumantes de type émulsion huile-dans-eau

Les compositions A à F de l'exemple 3 sont constituées des ingrédients suivants :

| **Composition Finale** | | **A** | **B** | **C** | **D** | **E** | **F** |
|---|---|---|---|---|---|---|---|
| **Nom** | **Nom INCI** | **% w/w final** | | | | | |
| **PHASE GEL AQUEUX** | **sous total** | 92,00 | | | | | |
| Eau osmosée | Aqua | 73.45 | | | | | |
| Microcare PE | Phenoxyethanol, aqua | 0.8 | | | | | |
| Microcare emollient PTG | Pentylene glycol, aqua | 2,00 | | | | | |
| Glycerine codex (99%) | Glycerin, aqua | 8 | | | | | |
| Zemea propanediol | Propanediol, aqua | 2 | | | | | |
| Butylene glycol 1,3 | Butvlene glycol, aqua | 1 | | | | | |
| EDETA bd | Disodium EDTA | 0,03 | | | | | |
| Carbopol ultrez 10 polymer | Carbomer | 0.4 | | | | | |
| Carbopol aqua SF-1 polymer | Aqua, Acrylates copolymer | 2,00 | | | | | |
| Hepes-luv | Hydroxyethylpiperazine ethane sulfonic acid, Aqua | 0,92 | | | | | |
| Sepimat sb | Methyl methacrylate crosspolymer, Aqua | 0.5 | | | | | |
| Orgasol 1002d nat cos | Nylon-6, Aqua | 0.5 | | | | | |
| Sodium hydroxide pellets prs codex | Sodium hydroxide | 0,40 | | | | | |
| **PHASE GRASSE** | **sous total** | 8,00 | 8,00 | 8,00 | 8,00 | 8,00 | 8,00 |
| DUB ININ Grade A | Isononyl isononanoate, Aqua | 4,98 | 4,96 | 4,94 | 4,92 | 4,88 | 4,976 |
| **TINOGARD TT** | Pentaerythrityl tetra-dit-butyl hvdroxvhvdrocinnamate | **0,00** | **0,02** | **0,04** | **0,06** | **0,10** | **0,004** |
| Parfum | Fragance | 3,00 | 3,00 | 3,00 | 3,00 | 3,00 | 3,00 |
| CAS-3131 PILOT | Amodimethicone | 0,02 | 0,02 | 0,02 | 0,02 | 0,02 | 0,02 |
| **TOTAL** | | *100,00* | *100,00* | *100,00* | *100,00* | *100,00* | *100,00* |

Le protocole de préparation de la composition A est similaire à celui décrit dans l'exemple 1.

Le protocole de préparation des compositions B à F est similaire à celui décrit dans l'exemple 1, à la différence que la préparation de la phase grasse (ou phase parfumante) comprend une étape de chauffage (environ 45°C) avant ajout du Tinogard TT dans le DUB ININ afin de solubiliser cet antioxydant de manière satisfaisante.

Ces compositions A à F sont ensuite placées chacune dans un dispositif de conditionnement selon l'invention (i.e. de type « cushion cream »).

Avec une composition A (i.e. selon l'invention mais dénuée d'antioxydant), les inventeurs ont observé l'apparition d'un léger jaunissement de cette dernière 15 jours après sa fabrication.

L'ajout d'un antioxydant (i.e. TINOGARD TT) a donc été envisagé dans des concentrations différentes (voir compositions B à F).

Des tests de stabilité, notamment au niveau du jaunissement, des compositions A à F ont été réalisés pendant 1 mois, à température ambiante (TA) et à 45°C.

Le jaunissement est identifié sur la base d'une observation visuelle et l'intensité de ce jaunissement est identifiée selon la barème suivant :

| **Valeur** | **Intensité du jaunissement de la composition** |
|---|---|
| **0** | Absence de jaunissement (composition blanche) |
| **1** | Léger |
| **2** | Moyen |
| **3** | Elevé |
| **4** | Très élevé |

Une observation visuelle des compositions A à F, à TA et 45°C, est effectuée une fois par semaine.

### Résultats :

| **Temps** (en semaines) | **A** | | **B** | | **C** | | **D** | | **E** | | **F** | |
|---|---|---|---|---|---|---|---|---|---|---|---|---|
| | **TA** | **45°C** | **TA** | **45°C** | **TA** | **45°C** | **TA** | **45°C** | **TA** | **45°C** | **TA** | **45°C** |
| 0 | 0 | 0 | 0 | 0 | 0 | 0 | 0 | 0 | 0 | 0 | 0 | 0 |
| 1 | 0 | 0 | 0 | 0 | 0 | 0 | 0 | 0 | 0 | 0 | 0 | 0 |
| 2 | 1 | 1 | 0 | 0 | 1 | 1 | 1 | 1 | 1 | 2 | 0 | 0 |
| 3 | 2 | 2 | 1 | 1 | 2 | 3 | 2 | 3 | 2 | 3 | 1 | 1 |
| 4 | 2 | 3 | 1 | 1 | 3 | 4 | 3 | 4 | 4 | 4 | 1 | 1 |

Un jaunissement de la composition A (dénuée d'antioxydant) est observé à partir de 2 semaines après fabrication de ladite composition A.

Un léger jaunissement est observé vers la fin de l'étude pour les compositions B et F.

En revanche, un jaunissement d'intensité supérieure des compositions C à E est observé à partir de 2 semaines après fabrication desdites compositions C à E, l'intensité de ce jaunissement étant proportionnelle à la concentration en antioxydant.

### Conclusion :

La mise en œuvre d'un antioxydant permet de réduire le phénomène de jaunissement d'une émulsion selon l'invention dans un intervalle de concentration particulier, à savoir :
- supérieur à 0% en poids par rapport au poids total de l'émulsion, et
- inférieur à 0,04% en poids par rapport au poids total de l'émulsion.

En effet, au-delà de 0,04% en poids d'antioxydant par rapport au poids total de la composition, le TINOGARD TT a une action pro-oxydante non souhaitée.

### Exemple 4 : Préparation d'une « Cushion cream » avec une composition parfumante de type émulsion huile-dans-eau obtenu via un procédé à chaud

La composition de l'exemple 4 est constituée des ingrédients suivants :

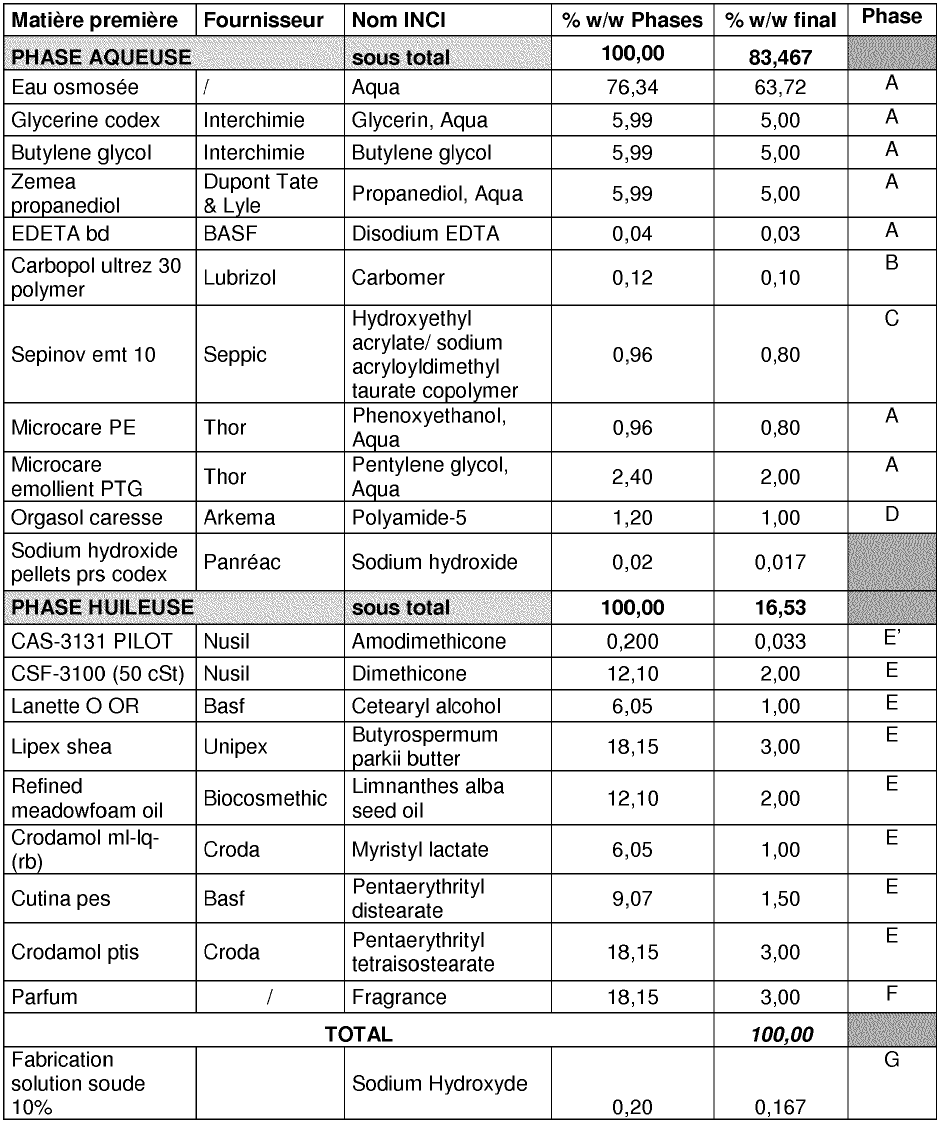

La composition de l'exemple 4 est préparée selon le protocole suivant :

### Préparation de la phase aqueuse :

Dans un premier réceptacle et à 60°C, ajouter sous agitation les matières premières A, puis ajouter au fur et à mesure sous agitation les matières premières B, C et D.

### Préparation de la phase grasse (phase parfumante) :

Dans un deuxième réceptacle et à 60°C, ajouter sous agitation les matières premières E, puis ajouter et homogénéiser la matière première E' (toujours à 60°C).

### Préparation de l'émulsion :

A 60°C, la phase parfumante est incorporée à la phase aqueuse sous forte agitation pendant 15 minutes.

Ensuite, on refroidit le mélange à 25°C puis on ajoute le parfum (F).

Enfin, on neutralise l'émulsion avec la solution de soude à 10% (G), et on agite jusqu'à obtenir un mélange homogène

Cette composition est ensuite placée dans un dispositif de conditionnement selon l'invention (i.e. de type « cushion cream ») ; dans le réceptacle dudit dispositif, la composition est imbibée/imprégnée au sein d'une éponge alvéolée (i.e. l'élément présentant au moins un orifice).

Le protocole d'application sur une matière kératinique et les résultats sont identiques à ceux décrits en exemple 1.

## Revendications

1. Dispositif de conditionnement comprenant au moins :
i) un réceptacle comprenant au moins un réservoir contenant au moins une émulsion comprenant une phase continue et une phase dispersée sous forme de gouttes, lesdites gouttes comprenant une écorce formée d'au moins un polymère cationique et d'au moins un polymère anionique, ladite émulsion comprenant au moins 2% en poids d'agent(s) parfumant(s) par rapport au poids total de ladite émulsion ;
ii) un moyen d'application de ladite émulsion sur une matière kératinique ; et
iii) un élément présentant au moins un orifice permettant le passage de ladite émulsion du réservoir vers ledit moyen d'application,
**caractérisé en ce que** l'élément comprenant au moins un orifice est choisi dans le groupe constitué d'une éponge, d'une plaque rigide percée d'au moins un trou, d'un filet et d'un tamis, et **en ce que** le moyen d'application est choisi dans le groupe constitué d'une brosse, d'une houppe, d'une houppette, d'une mousse, et d'un pinceau.

2. Dispositif selon la revendication 1, dans lequel l'élément comprenant au moins un orifice est une éponge alvéolée.

3. Dispositif selon l'une quelconque des revendications 1 ou 2, dans lequel l'émulsion est une émulsion huile-dans-eau.

4. Dispositif selon l'une quelconque des revendications 1 à 3, dans lequel le polymère cationique est un polymère silicone modifié par au moins une fonction amine primaire, secondaire ou tertiaire, tel que l'amodiméthicone.

5. Dispositif selon l'une quelconque des revendications 1 à 4, dans lequel chaque goutte comprend de 0,01% à 10% en poids de polymère(s) cationique(s) par rapport au poids total de la phase comprenant le(s)dit(s) polymère(s) cationique(s).

6. Dispositif selon l'une quelconque des revendications 1 à 5, dans lequel le polymère anionique est un carbomère ou un copolymère réticulé acrylates/C₁₀₋₃₀ alkyl acrylate.

7. Dispositif selon l'une quelconque des revendications 1 à 6, dans lequel ladite émulsion comprend de 0,01% à 5% en poids de polymère(s) anionique(s) par rapport au poids total de ladite émulsion.

8. Dispositif selon l'une quelconque des revendications 1 à 7, dans lequel l'émulsion comprend en outre au moins un polymère réticulé ou au moins un copolymère réticulé, ledit polymère réticulé ou copolymère réticulé comprenant au moins une unité dérivée de la polymérisation d'un des monomères choisi dans le groupe constitué de l'acide acrylique et/ou de l'acide méthacrylique et/ou de l'acrylate d'alkyle comprenant de 1 à 30 atomes de carbone, ou leurs sels.

9. Dispositif selon la revendication 8, dans lequel l'émulsion comprend de 0,1% à 10% en poids de polymère(s) réticulé(s) ou copolymère(s) réticulé(s) par rapport au poids total de ladite émulsion.

10. Dispositif selon l'une quelconque des revendications 1 à 9 dans lequel l'émulsion comprend de la glycérine en une teneur supérieure ou égale à 5% en poids par rapport au poids total de ladite émulsion.

11. Dispositif selon l'une quelconque des revendications 1 à 10, dans lequel l'émulsion comprend en outre au moins un actif choisi parmi les agents hydratants, les agents cicatrisants, les agents dépigmentants, les filtres UV, les agents desquamants, les agents antioxydants, les actifs stimulant la synthèse des macromoléculaires dermiques et/ou épidermiques, les agents dermodécontractants, les agents anti-transpirants, les agents apaisants, les agents anti-âge et leurs mélanges.

12. Dispositif selon l'une quelconque des revendications 1 à 11, dans lequel ladite émulsion ne comprend pas de tensioactif.

13. Dispositif selon l'une quelconque des revendications 1 à 12 dans lequel l'émulsion est sous forme d'une composition.

14. Procédé cosmétique non thérapeutique de maquillage et/ou de soin d'une matière kératinique et/ou pour améliorer la rétention de parfum sur une matière kératinique comprenant au moins les étapes suivantes :
i) mettre en contact le moyen d'application avec une émulsion du dispositif selon l'une quelconque des revendications 1 à 13 ; et
ii) appliquer l'émulsion présente sur ledit moyen d'application, sur ladite matière kératinique.

15. Procédé cosmétique non thérapeutique de maquillage et/ou de soin d'une matière kératinique et/ou pour améliorer la rétention de parfum sur une matière kératinique comprenant au moins les étapes suivantes :
i) mettre en contact le moyen d'application tel que défini dans la revendication 1, avec une émulsion contenue dans un dispositif selon l'une quelconque des revendications 1 à 13, ledit dispositif comprenant une éponge alvéolée à titre d'élément comprenant au moins un orifice ; et
ii) appliquer l'émulsion présente sur ledit moyen d'application, sur ladite matière kératinique.

16. Procédé cosmétique non thérapeutique de maquillage et/ou de soin d'une matière kératinique et/ou pour améliorer la rétention de parfum sur une matière kératinique selon la revendication 15, dans lequel le moyen d'application est une houppette.

## Patentansprüche

1. Verpackungsvorrichtung, umfassend mindestens:
i) ein Behältnis mit mindestens einen Behälter, der mindestens eine Emulsion enthält, umfassend eine kontinuierliche Phase und eine dispergierte Phase in Form von Tropfen, die Tropfen umfassend eine Schale aus mindestens einem kationischen Polymer und mindestens einem anionischen Polymer, die Emulsion umfassend mindestens 2 Gewichtsprozent Duftstoff(e), bezogen auf das Gesamtgewicht der Emulsion;
ii) eine Applikationseinrichtung zum Auftragen der Emulsion auf ein Keratinmaterial; und
iii) ein Element, das mindestens eine Öffnung aufweist, die den Durchgang der Emulsion aus dem Behälter zu der Applikationseinrichtung ermöglicht,
**dadurch gekennzeichnet, dass** das Element mindestens eine Öffnung umfasst, ausgewählt aus der Gruppe, bestehend aus einem Schwamm, einer starren Platte, die mit mindestens einem Loch perforiert ist, einem Netz und einem Sieb, und dass die Applikationseinrichtung ausgewählt ist aus der Gruppe, bestehend aus einer Bürste, einer Quaste, einer Puderquaste, einem Schaum und einem Pinsel.

2. Vorrichtung nach Anspruch 1, wobei das Element, das mindestens eine Öffnung umfasst, ein wabenförmiger Schwamm ist.

3. Vorrichtung nach einem der Ansprüche 1 oder 2, wobei die Emulsion eine Ölin-Wasser-Emulsion ist.

4. Vorrichtung nach einem der Ansprüche 1 bis 3, wobei das kationische Polymer ein Silikonpolymer ist, das durch mindestens eine primäre, sekundäre oder tertiäre Aminfunktion modifiziert ist, wie z. B. Amodimethicon.

5. Vorrichtung nach einem der Ansprüche 1 bis 4, wobei jeder Tropfen 0,01 bis 10 Gewichtsprozent kationische(s) Polymer(e) umfasst, bezogen auf das Gesamtgewicht der Phase, die das/die kationische(n) Polymer(e) umfasst.

6. Vorrichtung nach einem der Ansprüche 1 bis 5, wobei das anionische Polymer ein Carbomer oder ein vernetztes Copolymer aus Acrylaten/C₁₀₋₃₀-Alkylacrylat ist.

7. Vorrichtung nach einem der Ansprüche 1 bis 6, wobei die Emulsion 0,01 Gewichtsprozent bis 5 Gewichtsprozent anionische(s) Polymer(e), bezogen auf das Gesamtgewicht der Emulsion, umfasst.

8. Vorrichtung nach einem der Ansprüche 1 bis 7, wobei die Emulsion ferner mindestens ein vernetztes Polymer oder mindestens ein vernetztes Copolymer umfasst, wobei das vernetzte Polymer oder vernetzte Copolymer mindestens eine Einheit umfasst, die von der Polymerisation eines der Monomere abgeleitet ist, ausgewählt aus der Gruppe, bestehend aus Acrylsäure und/oder Methacrylsäure und/oder Alkylacrylat umfassend 1 bis 30 Kohlenstoffatome, oder deren Salzen.

9. Vorrichtung nach Anspruch 8, wobei die Emulsion 0,1 bis 10 Gewichtsprozent vernetzte(s) Polymer(e) oder vernetzte(s) Copolymer(e), bezogen auf das Gesamtgewicht der Emulsion, umfasst.

10. Vorrichtung nach einem der Ansprüche 1 bis 9, wobei die Emulsion Glycerin in einem Gehalt von 5 Gewichtsprozent oder mehr, bezogen auf das Gesamtgewicht der Emulsion, umfasst.

11. Vorrichtung nach einem der Ansprüche 1 bis 10, wobei die Emulsion ferner mindestens einen Wirkstoff umfasst, ausgewählt aus feuchtigkeitsspendenden Mitteln, wundheilenden Mitteln, depigmentierenden Mitteln, UV-Filtern, schuppenlösenden Mitteln, antioxidativen Mitteln, die Synthese der dermalen und/oder epidermalen Makromoleküle stimulierenden Wirkstoffen, dermo-dekontrahierenden Mitteln, Antitranspirantien, beruhigenden Mitteln, Anti-Aging-Mitteln und Gemischen davon.

12. Vorrichtung nach einem der Ansprüche 1 bis 11, wobei die Emulsion kein Tensid umfasst.

13. Vorrichtung nach einem der Ansprüche 1 bis 12, wobei die Emulsion in Form einer Zusammensetzung ist.

14. Kosmetisches, nicht therapeutisches Verfahren zum Schminken und/oder zur Pflege eines Keratinmaterials und/oder zum Verbessern der Rückhaltung von Parfüm auf einem Keratinmaterial, umfassend mindestens die folgenden Schritte:
i) Inkontaktbringen der Applikationseinrichtung mit einer Emulsion der Vorrichtung nach einem der Ansprüche 1 bis 13; und
ii) Auftragen der Emulsion, die auf der Applikationseinrichtung vorhanden, auf das Keratinmaterial.

15. Kosmetisches, nicht therapeutisches Verfahren zum Schminken und/oder zur Pflege eines Keratinmaterials und/oder zum Verbessern der Rückhaltung von Parfüm auf einem Keratinmaterial, umfassend mindestens die folgenden Schritte:
i) Inkontaktbringen der Applikationseinrichtung, wie definiert in Anspruch 1, mit einer Emulsion, die in einer Vorrichtung nach einem der Ansprüche 1 bis 13 enthalten ist, wobei die Vorrichtung einen wabenförmigen Schwamm als Element mit mindestens einer Öffnung umfasst; und
ii) Auftragen der Emulsion, die auf der Applikationseinrichtung vorhanden, auf das Keratinmaterial.

16. Kosmetisches, nicht therapeutisches Verfahren zum Schminken und/oder Pflegen eines Keratinmaterials und/oder zum Verbessern der Rückhaltung von Parfüm auf einem Keratinmaterial nach Anspruch 15, wobei die Applikationseinrichtung eine Puderquaste ist.

## Claims

1. Packaging device comprising at least:
i) a receptacle comprising at least one reservoir containing at least one emulsion comprising a continuous phase and a dispersed phase in the form of drops, wherein the drops comprise a shell formed by at least one cationic polymer and at least one anionic polymer, said emulsion comprising at least 2% by weight of perfuming agent(s) relative to the total weight of the emulsion;
ii) means for applying the emulsion to a keratin material; and
iii) an element having at least one orifice allowing the passage of the emulsion from the reservoir to the application means,
**characterized in that** the element comprising at least one orifice is selected from the group consisting of a sponge, a rigid plate pierced with at least one hole, a net and a sieve, and wherein the application means is selected from the group consisting of a brush, a puff, a pad, a foam, and a mini-brush.

2. Device according to claim 1, wherein the element comprising at least one orifice is a cellular sponge.

3. Device according to any one of claims 1 or 2, wherein the emulsion is an oil-in-water emulsion.

4. Device according to any one of claims 1 to 3, wherein the cationic polymer is a silicone polymer modified with at least one primary, secondary or tertiary amine function, such as amodimethicone.

5. Device according to any one of the claims 1 to 4, wherein each drop comprises from 0.01% to 10% by weight of cationic polymer(s) relative to the total weight of the phase comprising the cationic polymer(s).

6. Device according to any one of the claims 1 to 5, wherein the anionic polymer is a carbomer or a crosslinked copolymer acrylates/C₁₀₋₃₀ alkyl acrylate.

7. Device according to any one of claims 1 to 8, wherein the emulsion comprises from 0.01% to 5% by weight of anionic polymer(s) relative to the total weight of the emulsion.

8. Device according to any one of claims 1 to 7, wherein the emulsion further comprises at least one crosslinked polymer or at least one crosslinked copolymer, wherein the crosslinked polymer or crosslinked copolymer comprises at least one unit derived from the polymerization of at least one of the monomers selected from the group consisting of acrylic acid and/or methacrylic acid and/or alkyl acrylate with 1 to 30 carbon atoms, or their salts.

9. Device according to claim 8, wherein the emulsion comprises from 0.1% to 10% by weight of crosslinked polymer or crosslinked copolymer(s) relative to the total weight of the emulsion.

10. Device according to any one of claims 1 to 9 wherein the emulsion comprises glycerin in a content greater than or equal to 5% by weight relative to the total weight of the emulsion.

11. Device according to any one of claims 1 to 10, wherein the emulsion further comprises at least one active agent selected from among hydrating agents, healing agents, depigmenting agents, UV filters, desquamating agents, antioxidant agents, active agents stimulating the synthesis of dermal and/or epidermal macromoleculars, dermo-decontracting agents, antiperspirants, soothing agents, anti-aging agents and their mixtures.

12. Device according to any one of claims 1 to 11, wherein the emulsion does not include surfactant.

13. Device according to any one of claims 1 to 12 wherein the emulsion is in the form of a composition.

14. Non-therapeutic cosmetic method for making up and/or caring for a keratin material and/or for improving perfume retention on a keratin material comprising at least the following steps:
i) bringing the application means into contact with an emulsion of the device according to any one of claims 1 to 13; and
ii) applying the emulsion present on the application means to the keratin material.

15. Non-therapeutic cosmetic method for making up and/or caring for a keratin material and/or for improving perfume retention on a keratin material comprising at least the following steps:
i) bringing the application means according to claim 1, with an emulsion contained in the device according to any one of claims 1 to 13, said device comprising a cellular sponge as element comprising at least one orifice; and
ii) applying the emulsion present on the application means to the keratin material.

16. Non-therapeutic cosmetic method for making up and/or caring for a keratin material and/or for improving perfume retention on a keratin material according to claim 15, wherein the application means is a pad.
